# EUROPEAN PATENT APPLICATION

(11) **EP 2 581 368 A1**
(43) Date of publication of application: **17.04.2013**
(21) Application number: 11792581.8
(22) Date of filing: 06.06.2011
(51) Int. Cl.: C07D 273/08, C07D 498/10, C08G 85/00

(54) **CYCLIC CARBODIIMIDE COMPOUND**

(30) Priority: 16.06.2010 JP 2010137330; 10.06.2010 JP 2010132926
(71) Applicant: Teijin Limited, Osaka-shi, Osaka 541-0054 (JP)
(72) Inventor: SHOJI, Shinichiro, Iwakuni-shi Yamaguchi 740-0014 (JP)
(74) Representative: Carpmaels & Ransford
(86) International application number: PCT/JP2011/063430
(87) International publication number: WO 2011/155624

(57) **Abstract**

Disclosed is a cyclic carbodiimide compound useful as an end-capping agent for polymer compounds. The cyclic carbodiimide compound is represented by the following formula (i): 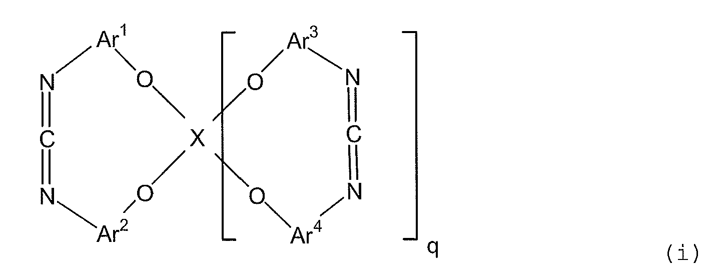 wherein X is a specific divalent group or tetravalent group, q is 0 when X is a divalent group, while q is 1 when X is a tetravalent group, and Ar¹ to Ar⁴ are selected from aromatic groups each independently optionally substituted with a group that serves as X.

## Description

### Technical Field

The present invention relates to a carbodiimide compound. More specifically, the invention relates to a cyclic carbodiimide compound.

### Background Art

Polyesters, polyamides, polyimides, polycarbonates, polyurethanes, and the like have excellent mechanical physical properties and thus have been used for a wide variety of applications. These polymers have a hydrolyzable ester bond, amide bond, imide bond, carbonate bond, or urethane bond in the molecule. Accordingly, when they are used in a more severe environment, a problem with reliability may occur, against which urgent countermeasures have been demanded.

The catalytic hydrolysis of a hydrolyzable bond such as an ester bond is promoted by the presence of a polar group such as a carboxyl group in the molecule. Therefore, a method for suppressing such a disadvantage by applying a carboxyl-group-capping agent to reduce the carboxyl group concentration has been proposed (Patent Document 1 and Patent Document 2).

As a capping agent for carboxyl groups and like acidic groups, a mono- or polycarbodiimide compound has been used considering the stability and reactivity of the capping agent, the color tone of the resulting product, and the like, and this has been effective to a certain degree. However, mono- and polycarbodiimide compounds are both linear carbodiimide compounds and thus have an intrinsic defect in that when they are used, a volatile isocyanate compound is by-produced, generating an offensive odor, whereby the working environment is deteriorated. There is a demand for the development of a capping agent that is free of such a defect and has higher reactivity.

Patent Document 3 describes a macrocyclic carbodiimide compound having a urethane bond and a polymer chain with a molecular weight of 100 to 7,000. Macrocyclic carbodiimide compounds have high molecular weight and thus are inefficient as acidic-group-capping agents. In addition, the prevention of an offensive odor is not considered in Patent Document 3.
[Patent Document 1] JP-A-2004-332166
[Patent Document 2] JP-A-2005-350829
[Patent Document 3] WO 2008/081230

### Disclosure of the Invention

An object of the inveniton is to provide a cyclic carbodiimide compound useful as a stabilizer for polymers having a hydrolyzable functional group, such as polyesters. Another object of the inveniton is to provide a method for producing the cyclic carbodiimide compound. Another object of the inveniton is to provide an end-capping agent for polymer compounds, which contains the cyclic carbodiimide compound as an active ingredient. Still another object of the inveniton is to provide an acidic group scavenger, which contains the cyclic carbodiimide compound as an active ingredient.

### Means for Solving the Problems

The present inventors conducted extensive research on capping agents whose reaction with an acidic group, such as a carboxyl group, does not causes the release of an isocyanate compound. As a result, they have found that the reaction of a carbodiimide compound having a ring structure with an acidic group does not cause the release of an isocyanate compound, whereby an offensive odor is not generated and the working environment is not deteriorated. The invention has thus been accomplished.

That is, the invention includes the following inventions.
1. A cyclic carbodiimide compound represented by the following formula (i): 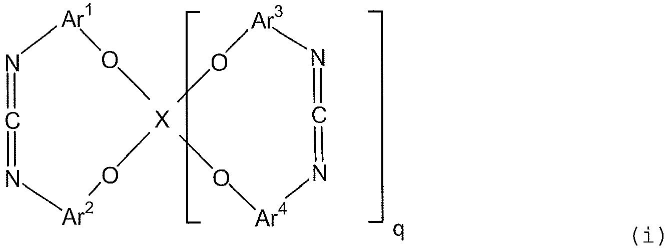 wherein
   X is a divalent group represented by any one of the following formulae (i-1) to (i-6) or a tetravalent group represented by any one of the following formulae (i-7) and (i-8) ,
   when X is a divalent group, q is 0, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6) Ar¹ and Ar² are each independently an aromatic group optionally substituted with a substituent, and
   when X is a tetravalent group, q is 1, and Ar¹ to Ar⁴ are each independently an aromatic group substituted with a substituent other than a C₁₋₆ alkyl group and a phenyl group: 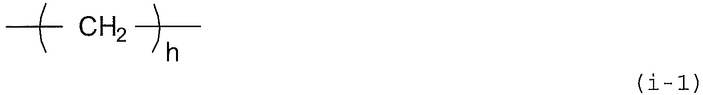 wherein h is an integer of 1 to 6, 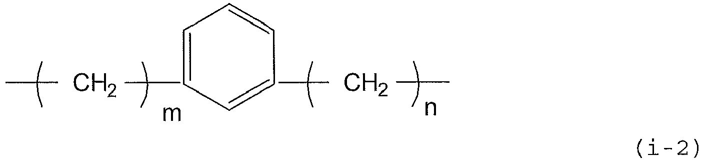 wherein m and n are each independently an integer of 0 to 3, 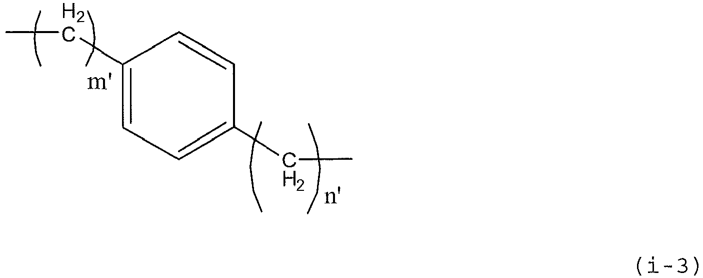 wherein m' and n' are each independently an integer of 0 to 3, 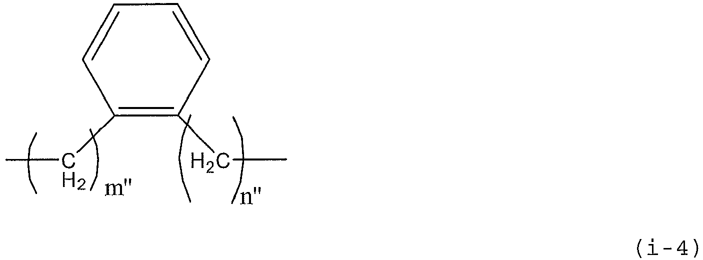 wherein m" and n" are each independently an integer of 0 to 3, 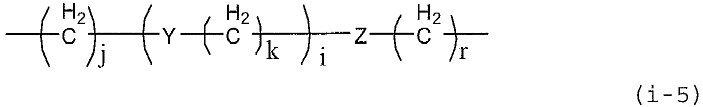 wherein Y and Z are each an oxygen atom or a sulfur atom, j, k, and r are each independently an integer of 1 to 4, and i is an integer of 0 to 3, 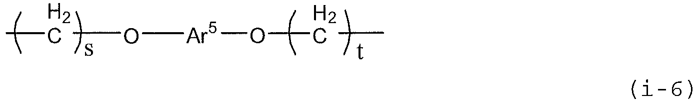 wherein Ar⁵ is an aromatic group, and s and t are each independently an integer of 1 to 3, 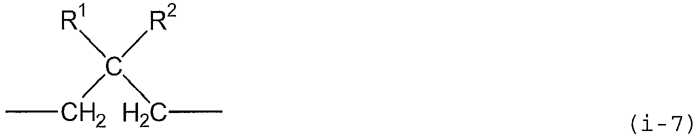 wherein R¹ and R² each independently represent a C₁₋₆ alkyl group or a phenyl group, 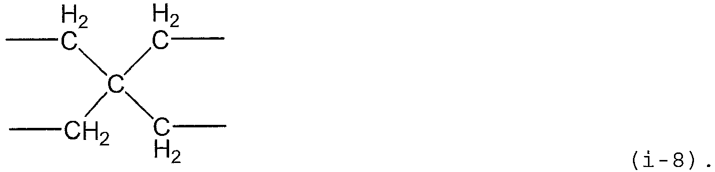
2. The compound according to the item 1 above, wherein Ar¹ to Ar⁴ are each independently an o-phenylene group or 1,2-naphthalene-diyl group substituted with a substituent other than a C₁₋₆ alkyl group and a phenyl group.
3. A method for producing the cyclic carbodiimide compound of the item 1 above, including:
   (1) a step (1a) of allowing a compound of the following formula (a-1) and a compound of the following formula (a-2) to react with a compound of the following formula (b-1) to give a nitro compound of the following formula (c):

      HO-Ar¹-NO₂ (a-1)

      HO-Ar²-NO₂ (a-2)

      E¹-X-E² (b-1)

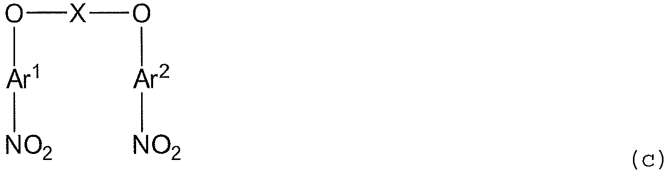

      wherein
      X, Ar¹, and Ar² are as defined in formula (i), with the proviso that X is a divalent group, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
      E¹ and E² are each independently a group selected from the group consisting of a halogen atom, a toluenesulfonyloxy group, a methanesulfonyloxy group, a benzenesulfonyloxy group, and a p-bromobenzenesulfonyloxy group;
   (2) a step (2a) of reducing the obtained nitro compound to give an amine compound represented by the following formula (d): 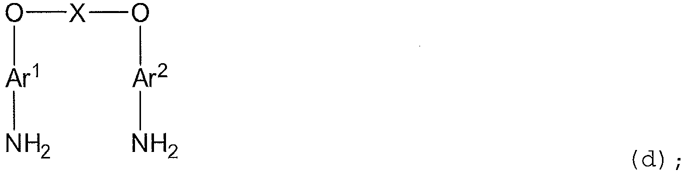
   (3) a step (3a) of allowing the obtained amine compound to react with triphenylphosphine dibromide to give a triphenylphosphine compound represented by the following formula (e-1): 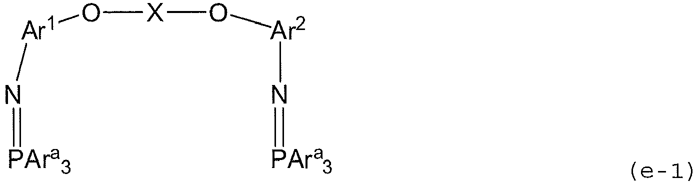 wherein Ar^{a} is a phenyl group; and
   (4) a step (4a) of isocyanating the obtained triphenylphosphine compound in a reaction system, followed by direct decarboxylation to give a compound of the following formula (f): 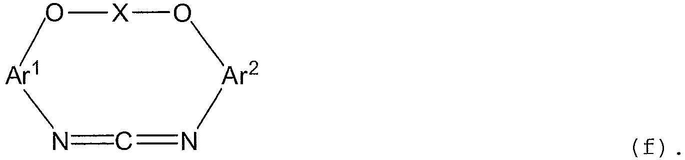
4. The method for producing the cyclic carbodiimide compound of the item 1 above according to the item 3 above, wherein the step (1a) is replaced with a step (1b) of allowing a compound of the following formula (a-i) and a compound of the following formula (a-ii) to react with a compound of the following formula (b-i):

   E³-Ar¹-NO₂ (a-i)

   E⁴-Ar²-NO₂ (a-ii)

   HO-X-OH (b-i)

   wherein
   X, Ar¹, and Ar² are as defined in formula (i), X is divalent, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
   E³ and E⁴ are each independently a group selected from the group consisting of a halogen atom, a toluenesulfonyloxy group, a methanesulfonyloxy group, a benzenesulfonyloxy group, and a p-bromobenzenesulfonyloxy group.
5. The method for producing the cyclic carbodiimide compound of the item 1 above according to the item 3 above, wherein
   the step (3a) is replaced with a step (3b) of allowing the amine compound to react with carbon dioxide or carbon disulfide to give a urea compound or thiourea compound represented by the following formula (e-2): 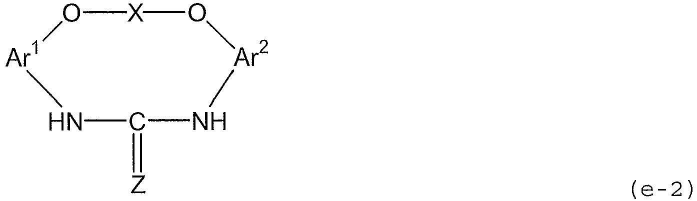 wherein
   X, Ar¹, and Ar² are as defined in formula (i), X is divalent, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
   Z is an oxygen atom or a sulfur atom, and the step (4a) is replaced with a step (4b) of dehydrating the obtained urea compound or desulfurizing the obtained thiourea compound.
6. A method for producing the cyclic carbodiimide compound of the item 1 above, including:
   (1) a step (1A) of allowing a compound of any one of the following formulae (A-1) to (A-4) to react with a compound of the following formula (B-1) to give a nitro compound of the following formula (C):

      HO-Ar¹-NO₂ (A-1)

      HO-Ar²-NO₂ (A-2)

      HO-Ar³-NO₂ (A-3)

      HO-Ar⁴-NO₂ (A-4)

      wherein
      Ar¹ to Ar⁴ are as defined in formula (i) and are each independently an aromatic group substituted with a substituent other than a C₁₋₆ alkyl group and a phenyl group, and
      E¹ to E⁴ are each independently a group selected from the group consisting of a halogen atom, a toluenesulfonyloxy group, a methanesulfonyloxy group, a benzenesulfonyloxy group, and a p-bromobenzenesulfonyloxy group, 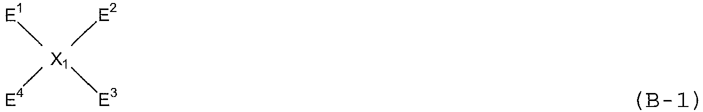 wherein X₁ is 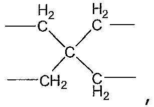 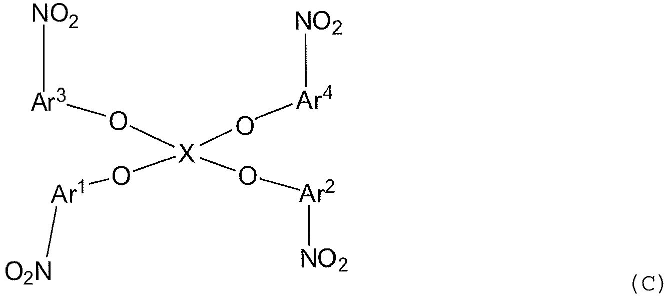 wherein X is as defined in formula (i), with the proviso that X is a tetravalent group represented by any one of formulae (i-7) and (i-8);
   (2) a step (2A) of reducing the obtained nitro compound to give an amine compound of the following formula (D): 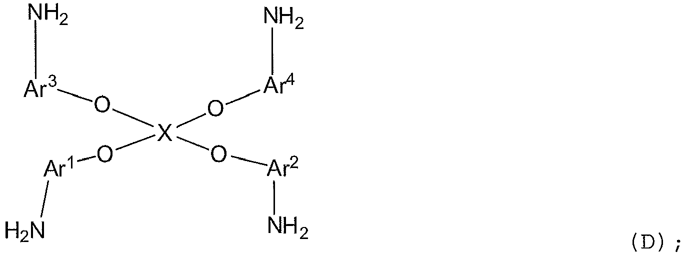
   (3) a step (3A) of allowing the obtained amine compound to react with triphenylphosphine dibromide to give a triphenylphosphine compound of the following formula (E-1): 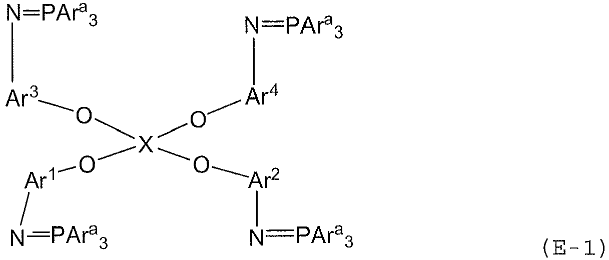 wherein Ar^{a} is a phenyl group; and
   (4) a step (4A) of isocyanating the obtained triphenylphosphine compound in a reaction system, followed by direct decarboxylation to give a compound (F) of the following formula: 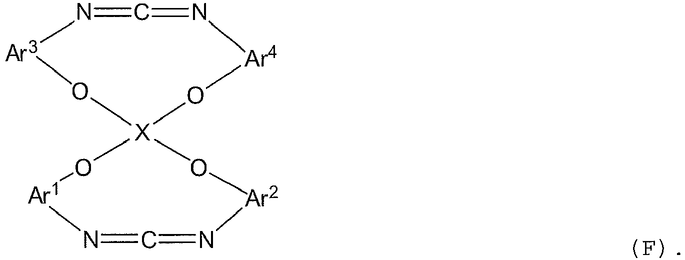
7. The method for producing the cyclic carbodiimide compound of the item 1 above according to the item 6 above, wherein the step (1A) is replaced with a step (1B) of allowing a compound of any one of the following formulae (A-i) to (A-iv) to react with a compound of the following formula (B-i) to give a nitro compound of formula (C):

   E⁵-Ar¹-NO₂ (A-i)

   E⁶-Ar²-NO₂ (A-ii)

   E⁷-Ar³-NO₂ (A-iii)

   E⁸-Ar⁴-NO₂ (A-iv)

   wherein
   Ar¹ to Ar⁴ are as defined in formula (i) and are each independently an aromatic group substituted with a substituent other than a C₁₋₆ alkyl group and a phenyl group, and
   E⁵ to E⁸ are each independently a group selected from the group consisting of a halogen atom, a toluenesulfonyloxy group, a methanesulfonyloxy group, a benzenesulfonyloxy group, and a p-bromobenzenesulfonyloxy group, 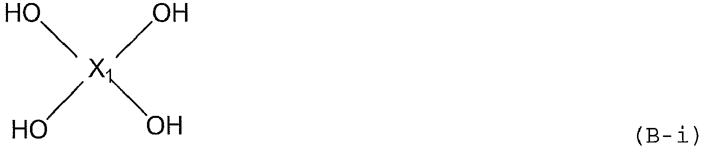 wherein X₁ is 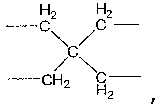 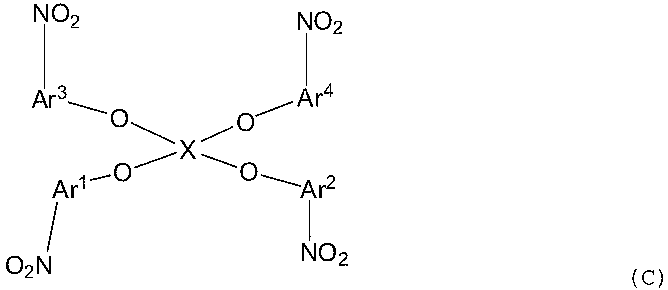 wherein X is as defined in formula (i), with the proviso that X is a tetravalent group represented by any one of formulae (i-7) and (i-8).
8. The method for producing the cyclic carbodiimide compound of the item 1 above according to the item 6 above, wherein
   the step (3A) is replaced with a step (3B) of allowing the amine compound to react with carbon dioxide or carbon disulfide to give a urea compound or thiourea compound of the following formula (E-2): 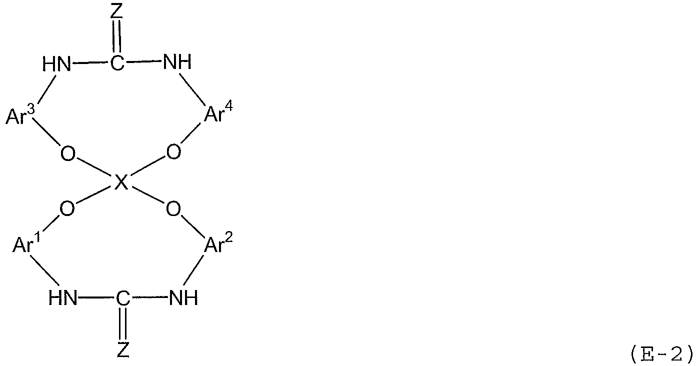 wherein
   Ar¹ to Ar⁴ are as defined in formula (i) and are each independently an aromatic group substituted with a substituent other than a C₁₋₆ alkyl group and a phenyl group,
   X is as defined in formula (i) and is a tetravalent group represented by any one of formulae (i-7) and (i-8), and
   Z is an oxygen atom or a sulfur atom, and
   the step (4A) is replaced with a step (4B) of dehydrating the obtained urea compound or desulfurizing the obtained thiourea compound.
9. An end-capping agent for polymer compounds, containing the cyclic carbodiimide compound represented by formula (i) of the item 1 above as an active ingredient.
10. An acidic group scavenger, containing the cyclic carbodiimide compound represented by formula (i) of the item 1 above as an active ingredient.

### Advantage of the Invention

The cyclic carbodiimide compound of the invention is capable of effectively stabilizing a hydrolyzable component of a polymer compound. At the same time, the by-production of a free isocyanate compound can also be suppressed. Even when the cyclic carbodiimide compound of the invention is used to end-cap a polymer compound, the generation of an offensive odor from an isocyanate compound can be suppressed, whereby the working environment is not deteriorated.

In addition, when a polymer compound is end-capped with the cyclic carbodiimide compound, isocyanate groups are produced at the ends of the polymer compound. The reaction of such isocyanate groups allows the molecular weight of the polymer compound to be increased.

In addition, the cyclic carbodiimide compound of the invention also has the function of scavenging free monomers or other acidic-group-containing compounds in the polymer compound.

Further, the cyclic carbodiimide compound of the invention has a ring structure and thus is advantageous in that ends can be capped under milder conditions as compared with linear carbodiimide compounds.

According to the production method of the invention, a cyclic carbodiimide can be easily produced. The cyclic carbodiimide compound of the invention is useful as an end-capping agent for polymer compounds. The cyclic carbodiimide compound of the invention is useful as an acidic group scavenger, particularly as a scavenger for free compounds in a polymer compound.

The difference in end-capping reaction mechanism between a linear carbodiimide compound and a cyclic carbodiimide compound is as follows.

When a linear carbodiimide compound (R¹-N=C=N-R²) is used as an end-capping agent for a polymer compound terminated with carboxyl groups, the reaction is as shown in the formula below. In the formula, W is the main chain of the polymer compound. Through the reaction of the linear carbodiimide compound with a carboxyl group, an amide group is formed at the end of the polymer compound, and an isocyanate compound (R¹NCO) is released.

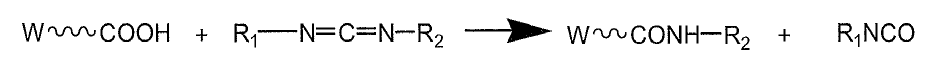

Meanwhile, when a cyclic carbodiimide compound is used as an end-capping agent for a polymer compound terminated with carboxyl groups, the reaction is as shown in the formula below. Through the reaction of the cyclic carbodiimide compound with a carboxyl group, an isocyanate group (-NCO) is formed at the end of the polymer compound via an amide group. It will be understood that no isocyanate compound is released.

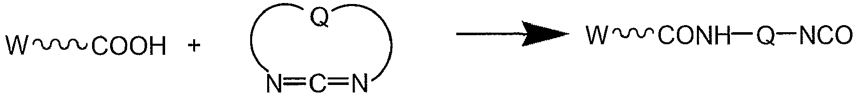

In the formula, Q is a divalent to tetravalent linking group that is an aliphatic group, an alicyclic group, an aromatic group, or a combination thereof and optionally contains a heteroatom and a substituent.

In addition, when two or more carbodiimides are present in one ring, this leads to a disadvantage in that an isocyanate compound is released during the carbodiimide group reaction.

### Mode for Carrying Out the Invention

Hereinafter, the invention will be described in detail.

### <Cyclic Carbodiimide Compound>

The invention is a cyclic carbodiimide compound represented by the following formula (i): 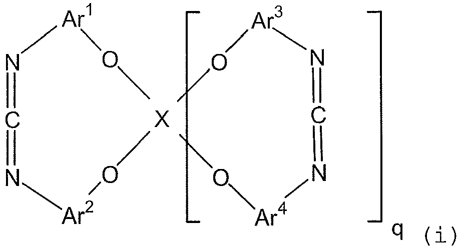 wherein
X is a divalent group represented by any one of the following formulae (i-1) to (i-6) or a tetravalent group represented by any one of the following formulae (i-7) and (i-8),
when X is a divalent group, q is 0, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
when X is a tetravalent group, q is 1, and Ar¹ to Ar⁴ are each independently an aromatic group substituted with a substituent other than a C₁₋₆ alkyl group and a phenyl group: 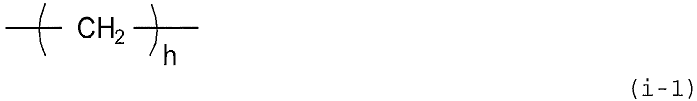 wherein h is an integer of 1 to 6, 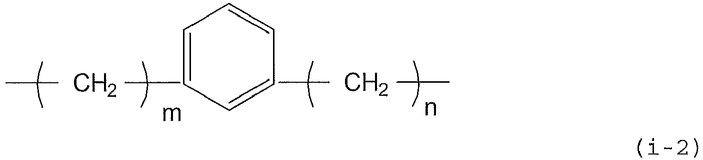 wherein m and n are each independently an integer of 0 to 3, 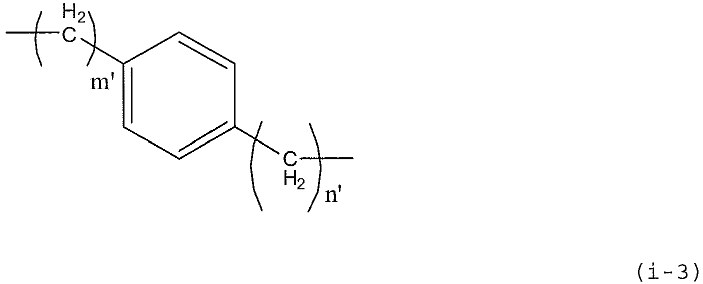 wherein m' and n' are each independently an integer of 0 to 3, 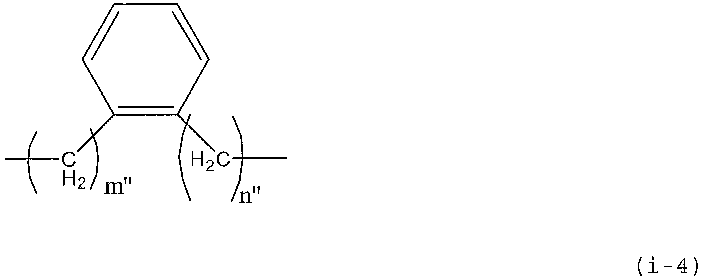 wherein m" and n" are each independently an integer of 0 to 3, 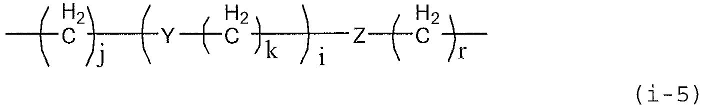 wherein Y and Z are each an oxygen atom or a sulfur atom, j, k, and r are each independently an integer of 1 to 4, and i is an integer of 0 to 3, 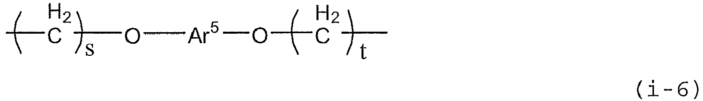 wherein Ar⁵ is an aromatic group, and s and t are each independently an integer of 1 to 3, 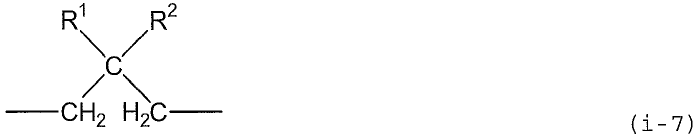 wherein R¹ and R² each independently represent a C₁₋₆ alkyl group or a phenyl group,

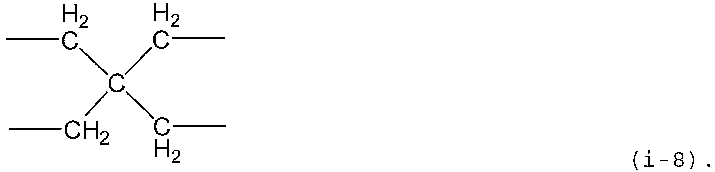

In the formula, when X is a divalent group selected from (i-1) and (i-2), Ar¹ and Ar² are each independently an aromatic group substituted with a substituent other than a C₁₋₆ alkyl group and a phenyl group. Examples of substituents other than a C₁₋₆ alkyl group and a phenyl group include an alkoxy group and a halogen group. Specific examples thereof include a methoxy group, an ethoxy group, a chloro group, and a fluoro group.

In the formula, when X is a divalent group selected from (i-3) to (i-6), Ar¹ and Ar² are each independently an optionally substituted aromatic group. In the case where they are substituted, a C₁₋₆ alkyl group, a phenyl group, and also conventionally known substituents are applicable as such substituents. Examples thereof include an alkyl group having 7 or more carbon atoms, an aryl group other than a phenyl group, an alkoxy group, a hydroxy group, an aldehyde group, an acyl group, a carboxyl group, an ester group, a nitro group, an amino group, a sulfo group, a sulfonyloxy group, a halogeno group, a silyl group, a vinyl group, an allyl group, a cyano group, an isonitrile group, an amide group, an imide group, and a thiol group. The substituent may also be a linking group to another polymer or cyclic carbodiimide compound. The presence of such a substituent is expected to be effective in increasing compatibility with a polymer such as a polyester and enhancing the effect of the cyclic carbodiimide compound of the invention. Its presence is also expected to be effective in suppressing the volatility of the cyclic carbodiimide compound.

In the formula, when X is a tetravalent group, Ar¹ to Ar⁴ are each independently an aromatic group substituted with a substituent other than a C₁₋₆ alkyl group and a phenyl group. Examples of substituents other than a C₁₋₆ alkyl group and a phenyl group include an alkoxy group and a halogen group. Specific examples thereof include a methoxy group, an ethoxy group, a chloro group, and a fluoro group.

Examples of aromatic groups include C₅₋₁₅ aromatic groups such as a phenylene group and a naphthalenediyl group.

X is a divalent or tetravalent group. When X is divalent, q is 0. When X is tetravalent, q is 1. It is preferable that X is a divalent group represented by the following formula (i-1).

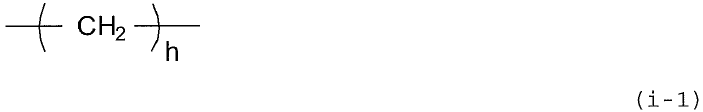

In the formula, h is an integer of 1 to 6. Preferred examples of groups represented by (i-1) include a methylene group, an ethylene group, a 1,3-propylene group, a 1,4-butylene group, a 1,5-pentane group, and a 1,6-hexane group. In a 1,3-propylene group, a 1,4-butylene group, a 1,5-pentane group, or a 1,6-hexane group, carbon that is not directly attached to oxygen may be substituted with at least one member selected from the group consisting of a C₁₋₆ alkyl group and a phenyl group. Examples of C₁₋₆ alkyl groups include a methyl group, an ethyl group, an n-propyl group, a sec-propyl group, an iso-propyl group, an n-butyl group, a tert-butyl group, a sec-butyl group, an isobutyl group, an n-pentyl group, a sec-pentyl group, an iso-pentyl group, an n-hexyl group, a sec-hexyl group, and an iso-hexyl group.

It is preferable that X is a group represented by the following formula (i-2).

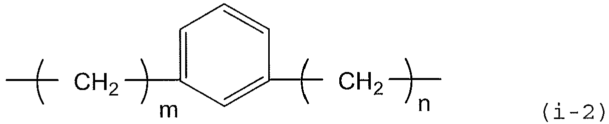

In the formula, m and n are each independently an integer of 0 to 3. When m = 0, the methylene group represents a single bond. When X has a 1,3-phenylene group, the stability of the cyclic carbodiimide compound of the invention is further enhanced, leading to an advantage in that a polymer compound can be applied at a higher process temperature.

It is preferable that X is a group represented by the following formula (i-3) and/or (i-4): 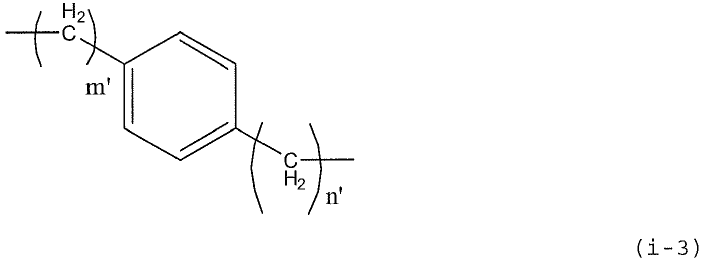 wherein m' and n' are each independently an integer of 0 to 3, 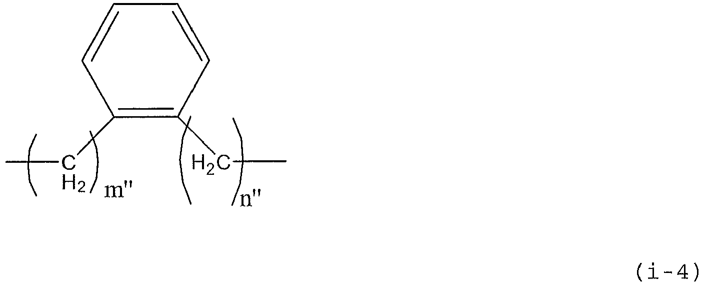 wherein m" and n" are each independently an integer of 0 to 3.

Here, when m' = 0 or m" = 0, the methylene group represents a single bond. When X has a phenylene group, the stability of the cyclic carbodiimide compound of the invention is further enhanced, leading to an advantage in that a polymer compound can be applied at a higher process temperature.

It is preferable that X is a group represented by the following formula (i-5).

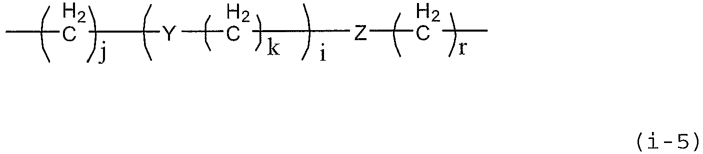

In the formula, Y and Z are each an oxygen atom or a sulfur atom, j, k, and r are each independently an integer of 1 to 4, and i is an integer of 0 to 3. The presence of an oxygen atom or a sulfur atom in X is expected to be effective in increasing compatibility with a polymer such as a polyester and enhancing the effect of the cyclic carbodiimide compound of the invention.

It is preferable that X is a divalent group represented by the following formula (i-4).

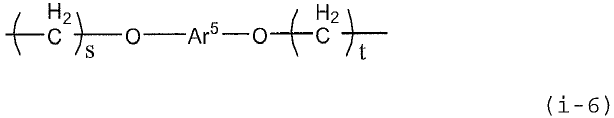

In the formula, Ar⁵ is an aromatic group, and s and t are each independently an integer of 1 to 3.

Examples of aromatic groups include an o-phenylene group, a m-phenylene group, and a p-phenylene group.

It is preferable that X is a group represented by the following formula (i-7).

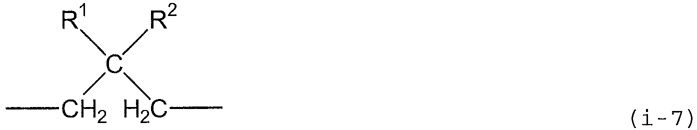

In the formula, R¹ and R² are each independently a C₁₋₆ alkyl group or a phenyl group. Examples of C₁₋₆ alkyl groups include a methyl group, an ethyl group, an n-propyl group, a sec-propyl group, an iso-propyl group, an n-butyl group, a tert-butyl group, a sec-butyl group, an iso-butyl group, an n-pentyl group, a sec-pentyl group, an iso-pentyl group, an n-hexyl group, a sec-hexyl group, and an iso-hexyl group.

It is preferable that X is a group represented by the following formula (i-8).

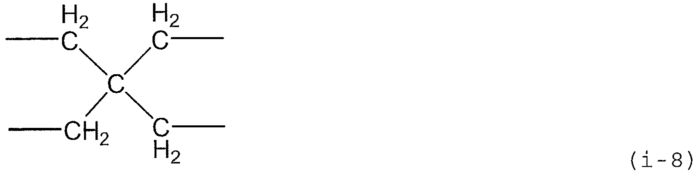

The cyclic carbodiimide compound of the invention may be a monocyclic compound of the following formula (f) or a bicyclic compound of the following formula (F).

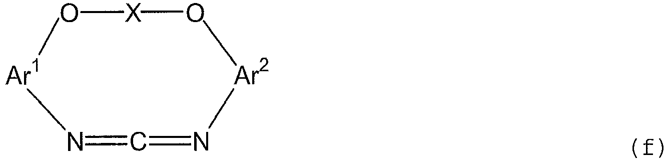

In the formula, Ar¹, Ar² and X are as defined in formula (i). It is preferable that Ar¹ and Ar² are each a substituted o-phenylene group. X is a divalent group.

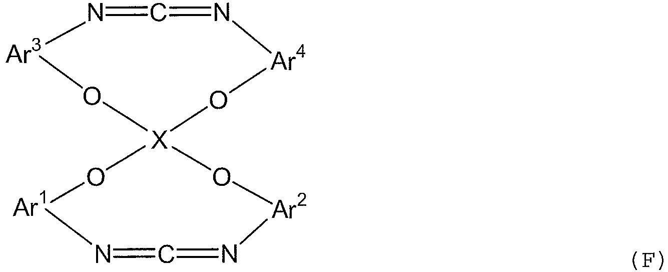

In the formula, Ar¹ to Ar⁴ and X are as defined in formula (i). It is preferable that Ar¹ to Ar⁴ are each a substituted o-phenylene group. X is a tetravalent group.

It is preferable that the cyclic carbodiimide compound of the invention has two o-phenylene groups at the 1- and 3-positions of the carbodiimide group, the o-phenylene groups each have ether oxygen at the ortho-position of the carbodiimide group, and the ether oxygen atoms are linked by X to form a ring structure.

That is, a compound represented by the following formula is preferable.

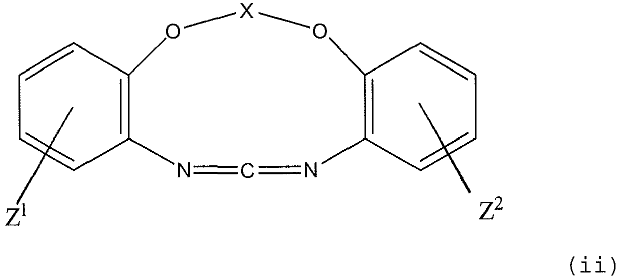

In the formula, X is as defined in formula (i) . Z¹ and Z² are each independently a substituent. Conventionally known substituents are applicable as such substituents, examples thereof including an alkyl group, an aryl group, an alkoxy group, a hydroxy group, an aldehyde group, an acyl group, a carboxyl group, an ester group, a nitro group, an amino group, a sulfo group, a sulfonyloxy group, a halogeno group, a silyl group, a vinyl group, an allyl group, a fluoroalkyl group, a cyano group, an isonitrile group, an amide group, an imide group, and a thiol group.

Examples of cyclic carbodiimide compounds according to the invention include the following compounds. 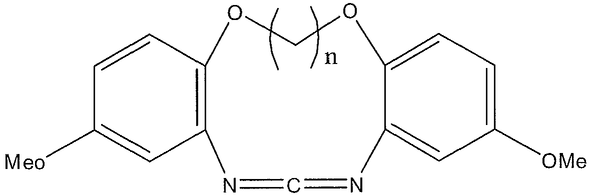 (n = an integer of 1 to 6) 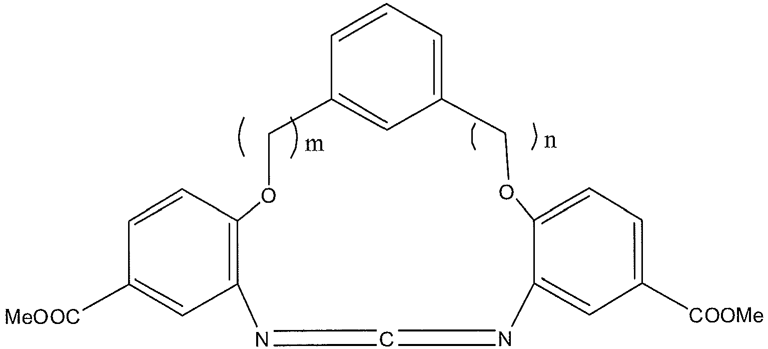 (m = an integer of 0 to 3, n = an integer of 0 to 3) 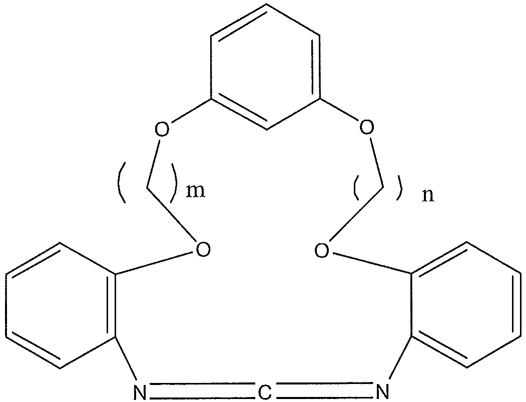 (m = an integer of 1 to 3, n = an integer of 1 to 3) 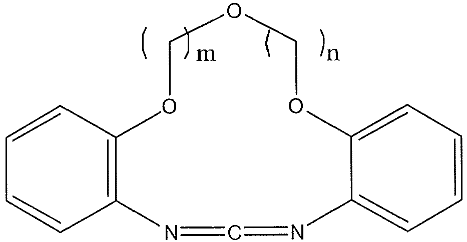 (m = an integer of 1 to 4, n = an integer of 1 to 4) 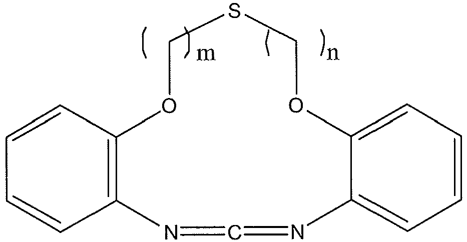 (m = an integer of 1 to 4, n = an integer of 1 to 4) 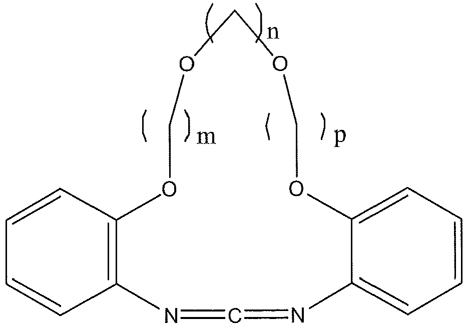 (m = an integer of 1 to 4, n = an integer of 1 to 4, p = an integer of 1 to 4) 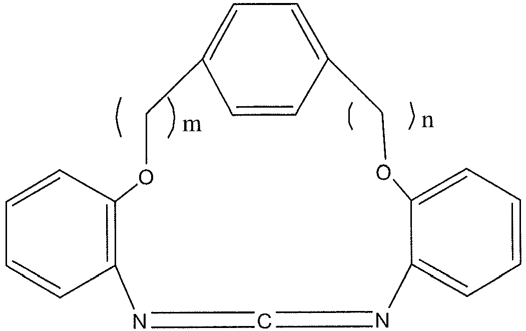 (m = an integer of 0 to 3, n = an integer of 0 to 3) 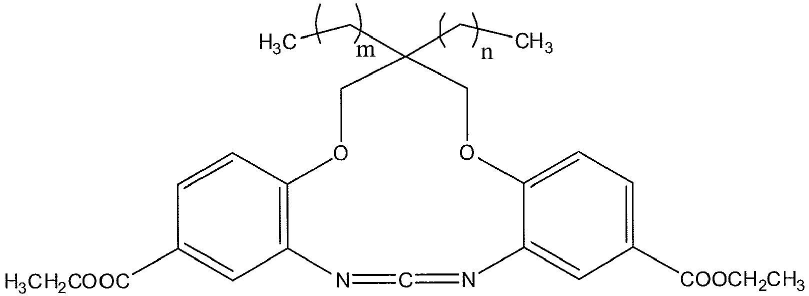 (m = an integer of 0 to 5, n = an integer of 0 to 5) 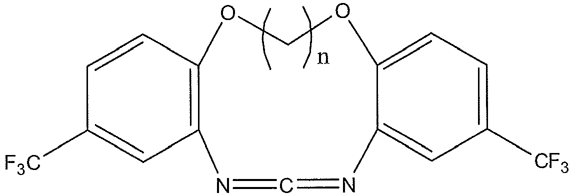 (n = an integer of 1 to 6)

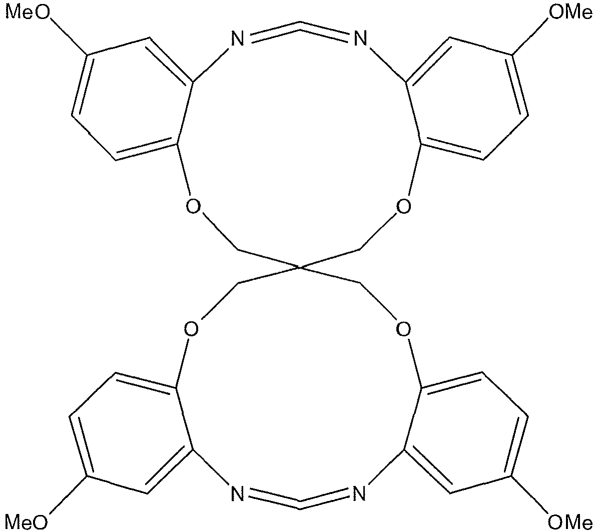

It is preferable that the cyclic carbodiimide compound of the invention has a molecular weight of 100 to 1,000. When the molecular weight is less than 100, this may cause problems with the structural stability or volatility of the cyclic carbodiimide compound. In addition, when the molecular weight is more than 1,000, in the production of the cyclic carbodiimide, synthesis in a dilution system may be required or the yield may decrease, causing problems with cost. From such a point of view, the molecular weight is more preferably 100 to 750, and still more preferably 250 to 750. The cyclic carbodiimide compound of the invention has one carbodiimide group in one ring. In the case where it has two or more carbodiimide groups in one ring, an isocyanate compound is formed during the end-capping reaction, causing an offensive odor.

### <Production of Monocyclic Carbodiimide Compound (f)>

The monocyclic carbodiimide compound (f) of the invention can be produced through the following steps (1) to (4).

The step (1) is a step for obtaining a nitro compound (c). The step (1) has two modes, a step (1a) and a step (1b). The step (2) is a step for obtaining an amide compound (d) from the nitro compound (c). The step (3) and the step (4) are steps for obtaining a monocyclic carbodiimide compound (f) from the amide compound (d). The steps (3) and (4) have a mode that goes through a step (3a) and a step (4a) and a mode that goes through a step (3b) and a step (4b).

Specifically, the carbodiimide compound (f) can be produced as follows.
(Scheme 1) : Step (1a)-step (2a)-step (3a)-step (4a)
(Scheme 2): Step (1a) -step (2a)-step (3b)-step (4b)
(Scheme 3): Step (1b)-step (2a)-step (3b)-step (4b)
(Scheme 4): Step (1b)-step (2a)-step (3a)-step (4a)

### (Step (1a))

The step (1a) is a step of allowing a compound of the following formula (a-1) and a compound of the following formula (a-2) to react with a compound of the following formula (b-1) to give a nitro compound (c) of the following formula.

HO-Ar¹-NO₂ (a-1)

HO-Ar²-NO₂ (a-2)

E¹-X-E² (b-1)

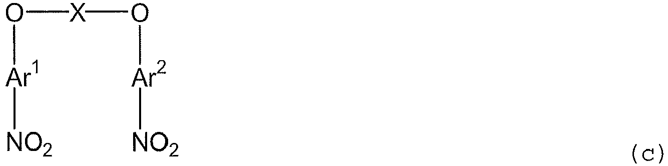

In the formulae, X, Ar¹, and Ar² are as defined in formula (i). X is a divalent group.

E¹ and E² are each independently a group selected from the group consisting of a halogen atom, a toluenesulfonyloxy group, a methanesulfonyloxy group, a benzenesulfonyloxy group, and a p-bromobenzenesulfonyloxy group. Examples of halogen atoms include a chlorine atom, a bromine atom, and an iodine atom.

The reaction may be a conventionally known ether synthesis method. For example, it is possible to use the Williamson reaction in which a compound represented by formula (a-1) and a compound represented by formula (a-2) are allowed to react with a compound represented by formula (b-1) in a solvent in the presence of a basic compound, etc.

Examples of basic compounds include sodium hydride, metallic sodium, sodium hydroxide, potassium hydroxide, and potassium carbonate. Examples of solvents include N,N-dimethylformamide, N-methyl-2-pyrrolidone, and tetrahydrofuran. The reaction temperature is suitably selected within a range of 25°C to 150°C. In addition, although the reaction proceeds rapidly enough under the above conditions, it is also possible to add a phase-transfer catalyst in order to promote the reaction.

### (Step (1b))

The step (1b) is a step of allowing a compound of the following formula (a-i) and a compound of the following formula (a-ii) to react with a compound of the following formula (b-i) to give a nitro compound of the following formula (c).

E³-Ar¹-NO₂ (a-i)

E⁴-Ar²-NO₂ (a-ii)

HO-X-OH (b-i)

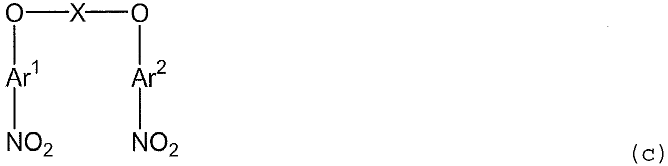

In the formulae, Ar¹, Ar², and X are as defined in formula (i). X is a divalent group. E³ and E⁴ are each independently a group selected from the group consisting of a halogen atom, a toluenesulfonyloxy group, a methanesulfonyloxy group, a benzenesulfonyloxy group, and a p-bromobenzenesulfonyloxy group.

The reaction may be a conventionally known ether synthesis method. For example, it is possible to use the Williamson reaction in which a compound represented by formula (a-i) and a compound represented by formula (a-ii) are allowed to react with a compound represented by formula (b-i) in a solvent in the presence of a basic compound, etc.

Examples of basic compounds include sodium hydride, metallic sodium, sodium hydroxide, potassium hydroxide, and potassium carbonate. Examples of solvents include N,N-dimethylformamide, N-methyl-2-pyrrolidone, and tetrahydrofuran. The reaction temperature is suitably selected within a range of 25°C to 150°C. In addition, although the reaction proceeds under the above conditions, it is preferable to add a phase-transfer catalyst in order to promote the reaction. Examples of phase-transfer catalysts include a tetrabutylammonium salt, a trioctylmethylammonium salt, a benzyldimethyloctadecylammonium salt, and crown ether.

### (Step (2))

The step (2) is a step of reducing the obtained nitro compound (c) to give an amine compound (d) of the following formula.

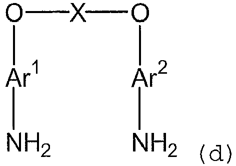

Ar¹, Ar², and X are as defined in formula (i). X is a divalent group.

The reaction may be a conventionally known method. For example, it is possible to use a method in which the nitro compound (c) is catalytically reduced in a solvent in the presence of hydrogen and a catalyst.

Examples of catalysts include palladium carbon, palladium carbon-ethylenediamine composites, palladium-fibroin, palladium-polyethyleneimine, nickel, and copper.

Examples of solvents include methanol, ethanol, isopropyl alcohol, dioxane, tetrahydrofuran, ethyl acetate, dichloromethane, chloroform, and N,N-dimethylformamide. The reaction temperature is suitably selected within a range of 25°C to 100°C. In addition, although the reaction proceeds at normal pressure, it is preferable to apply pressure in order to promote the reaction.

The reaction to produce an amine compound (d) may also be a method in which the nitro compound (c) is allowed to react with an acid and a metal, a method in which the nitro compound (c) is allowed to react with hydrazine and a catalyst, etc.

### (Step (3a))

The step (3a) is a step of allowing the obtained amine compound (d) to react with triphenylphosphine dibromide to give a triphenylphosphine compound (e-1) of the following formula.

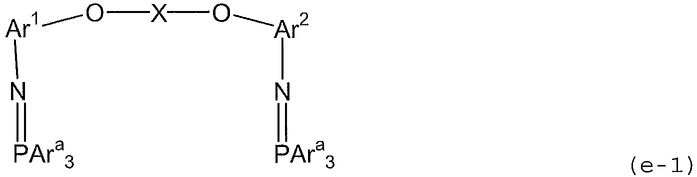

In the formula, Ar¹, Ar², and X are as defined in formula (i), and Ar^{a} is a phenyl group.

The reaction may be a conventionally known method. For example, it is possible to use a method in which the amine compound represented by formula (d) is allowed to react with triphenylphosphine dibromide in a solvent in the presence of a basic compound, etc. Examples of basic compounds include triethylamine and pyridine. Examples of solvents include dichloroethane, chloroform, and benzene. The reaction temperature is suitably selected within a range of 0°C to 80°C.

### (Step (4a))

The step (4a) is a step of isocyanating the obtained triphenylphosphine compound in a reaction system, followed by direct decarboxylation to give a cyclic carbodiimide compound (f).

The reaction may be a conventionally known method. For example, it is possible to use a method in which the triphenylphosphine compound of formula (e-1) is allowed to react in a solvent in the presence of di-tert-butyl dicarbonate and N,N-dimethyl-4-aminopyridine, etc. Examples of solvents include dichloromethane and chloroform. The reaction temperature is suitably selected within a range of 10°C to 40°C.

### (Step (3b))

The step (3b) is a step of allowing the amine compound (d) to react with carbon dioxide or carbon disulfide to give a urea compound or thiourea compound represented by the following formula (e-2).

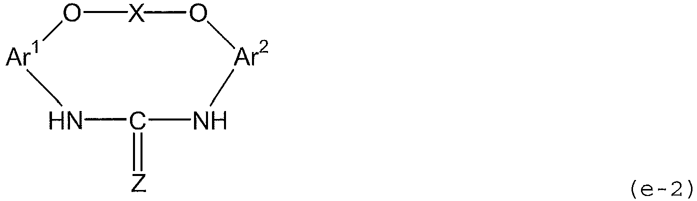

In the formula, Ar¹, Ar², and X are as defined in formula (i), and Z is an oxygen atom or a sulfur atom.

The reaction to produce a urea compound (e-2) may be a conventionally known method. For example, it is possible to use a method in which the amine compound (d) is allowed to react in a solvent in the presence of carbon dioxide, a phosphorus compound, and a basic compound.

Examples of phosphorus compounds include phosphite and phosphonate. Examples of basic compounds include triethylamine, pyridine, imidazole, and picoline.

Examples of solvents include pyridine, N,N-dimethylformamide, acetonitrile, chlorobenzene, and toluene. The reaction temperature is suitably selected within a range of 0°C to 80°C.

The reaction to produce a urea compound (e-2) may also be a method in which the amine compound (d) is allowed to react with carbon monoxide, a method in which the amine compound (d) is allowed to react with phosgene, etc.

The reaction to produce a thiourea compound (e-2) may be a conventionally known method. For example, it is possible to use a method in which the amine compound (d) is allowed to react in a solvent in the presence of carbon disulfide and a basic compound, etc.

Examples of basic compounds include triethylamine, pyridine, imidazole, and picoline. Examples of solvents include acetone, methanol, ethanol, isopropyl alcohol, 2-butanone, pyridine, N,N-dimethylformamide, and acetonitrile. The reaction temperature is suitably selected within a range of 25°C to 90°C. Although the reaction proceeds rapidly enough under the above conditions, it is also possible to use carbon tetrabromide or the like together in order to promote the reaction.

### (Step (4b))

The step (4b) is a step of dehydrating the obtained urea compound (e-2) or desulfurizing the obtained thiourea compound (e-2) to give a cyclic carbodiimide compound (f).

The reaction may be a conventionally known method. For example, it is possible to use a method in which the urea compound or thiourea compound (e-2) is allowed to react in a solvent in the presence of toluenesulfonyl chloride or methylsulfonyl chloride to dehydrate the urea compound (e-2) or desulfurize the thiourea compound (e-2).

Examples of solvents include dichloromethane, chloroform, and pyridine. The reaction temperature is suitably selected within a range of 0°C to 80°C.

The reaction to produce a cyclic carbodiimide compound (f) may also be a method in which the urea compound (e-2) is allowed to react with mercury oxide, a method in which the thiourea compound (e-2) is allowed to react with sodium hypochlorite, etc.

### <Production of Bicyclic Carbodiimide Compound (F)>

The bicyclic carbodiimide compound (F) of the invention can be produced through the following steps (1) to (4).

The step (1) is a step for obtaining a nitro compound (C). The step (1) has two modes, a step (1A) and a step (1B). The step (2) is a step for obtaining an amide compound (D) from the nitro compound (C). The step (3) and the step (4) are steps for obtaining a bicyclic carbodiimide compound (F) from the amide compound (D). The steps (3) and (4) have a mode that goes through a step (3A) and a step (4A) and a mode that goes through a step (3B) and a step (4B).

The carbodiimide compound (F) can be produced as follows.
(Scheme 1) Step (1A) - step (2A)-step (3A) - step (4A)
(Scheme 2) Step (1A) - step (2A)-step (3B) - step (4B)
(Scheme 3) Step (1B) - step (2A)-step (3B) - step (4B)
(Scheme 4) Step (1B) - step (2A)-step (3A)-step (4A)

### (Step (1A))

The step (1A) is a step of allowing a compound of any one of the following formulae (A-1) to (A-4) to react with a compound of the following formula (B-1) to give a nitro compound of the following formula (C).

HO-Ar¹-NO₂ (A-1)

HO-Ar²-NO₂ (A-2)

HO-Ar³-NO₂ (A-3)

HO-Ar⁴-NO₂ (A-4)

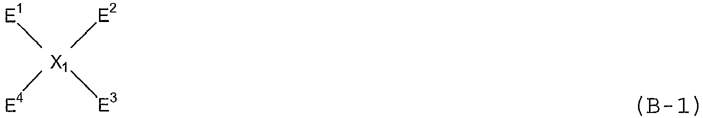

(X₁ is

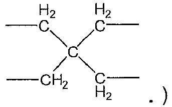

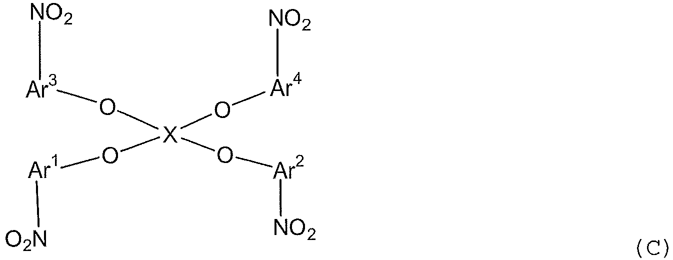

In the formulae, Ar¹ to Ar⁴ and X are as defined in formula (i). X is a tetravalent group. E¹ to E⁴ are each independently a group selected from the group consisting of a halogen atom, a toluenesulfonyloxy group, a methanesulfonyloxy group, a benzenesulfonyloxy group, and a p-bromobenzenesulfonyloxy group.

The reaction conditions are the same as in the step (1a) mentioned above.

### (Step (1B))

The step (1B) is a step of allowing a compound of any one of the following formulae (A-i) to (A-iv) to react with a compound of the following formula (B-i) to give a nitro compound of the following formula (C).

E⁵-Ar¹-NO₂ (A-i)

E⁶-Ar²-NO₂ (A-ii)

E⁷-Ar³-NO₂ (A-iii)

E⁸-Ar⁴-NO₂ (A-iv)

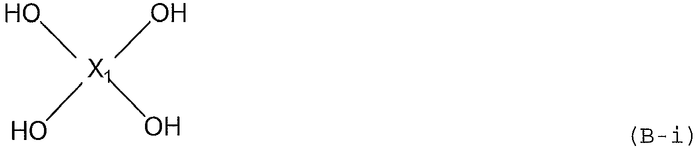

(X₁ is

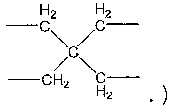

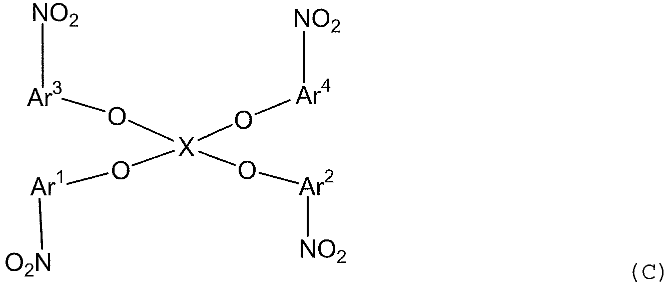

In the formulae, Ar¹ to Ar⁴ and X are as defined in formula (i). E⁵ to E⁸ are each independently a group selected from the group consisting of a halogen atom, a toluenesulfonyloxy group, a methanesulfonyloxy group, a benzenesulfonyloxy group, and a p-bromobenzenesulfonyloxy group.

The reaction conditions are the same as in the step (1b) mentioned above.

### (Step (2A))

The step (2A) is a step of reducing the obtained nitro compound to give an amine compound (D) of the following formula.

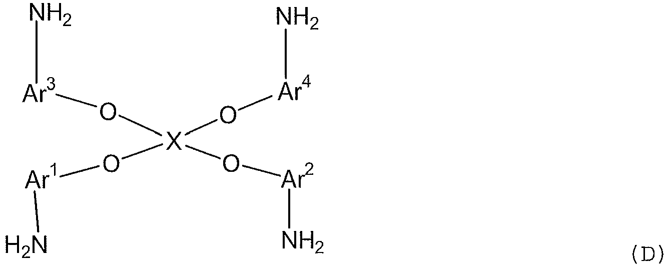

Ar¹ to Ar⁴ and X are as defined in formula (i).

The reaction conditions are the same as in the step (2a) mentioned above.

### (Step (3A))

The step (3A) is a step of allowing the obtained amine compound (D) to react with triphenylphosphine dibromide to give a triphenylphosphine compound (E-1) of the following formula.

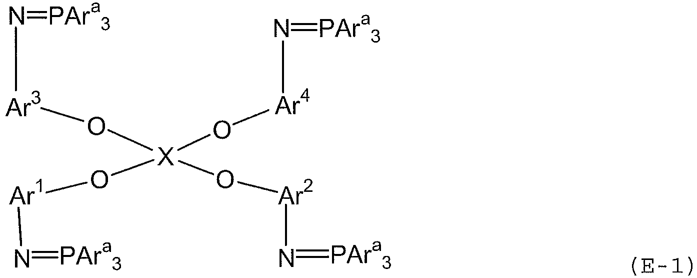

In the formula, Ar¹ to Ar⁴ and X are as defined in formula (i), and Ar^{a} is a phenyl group.

The reaction conditions are the same as in the step (3a) mentioned above.

### (Step (4A))

The step (4A) is a step of isocyanating the obtained triphenylphosphine compound in a reaction system, followed by direct decarboxylation to give a compound (F) of the following formula.

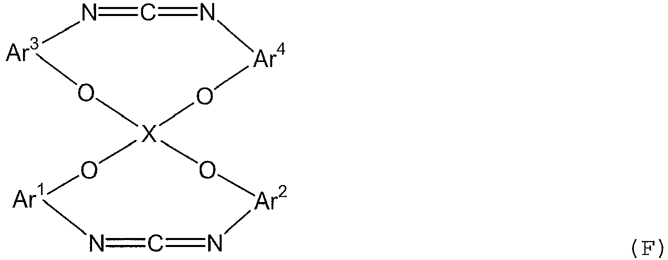

In the formula, Ar¹ to Ar⁴ and X are as defined in formula (i).

The reaction conditions are the same as in the step (4a) mentioned above.

### (Step (3B))

The step (3B) is a step of allowing the amine compound to react with carbon dioxide or carbon disulfide to give a urea compound or thiourea compound (E-2) of the following formula.

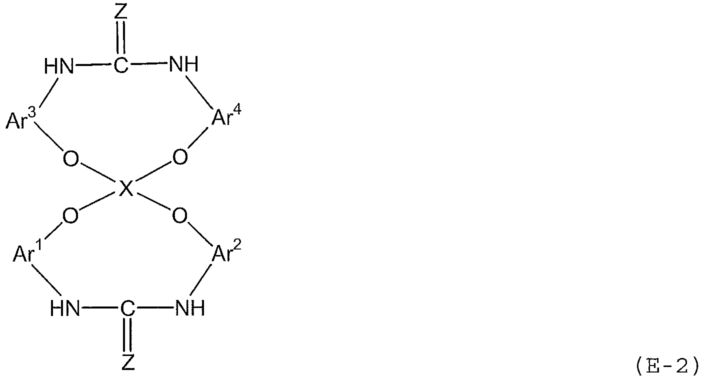

In the formula, Ar¹ to Ar⁴ and X are as defined in formula (i), and Z is an oxygen atom or a sulfur atom.

The reaction conditions are the same as in the step (3b) mentioned above.

### (Step (4B))

The step (4B) is a step of dehydrating the obtained urea compound or desulfurizing the obtained thiourea compound to give a compound (F) of the following formula.

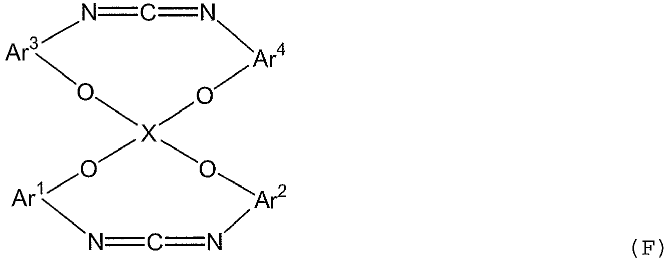

In the formula, Ar¹ to Ar⁴ and X are as defined in formula (i).

The reaction conditions are the same as in the step (4b) mentioned above.

### (Other Production Methods)

In addition to the above production methods, the cyclic carbodiimide compound of the invention can also be produced by conventionally known methods. Examples of methods include production from an amine compound via an isocyanate compound, production from an amine compound via an isothiocyanate compound, and production from a carboxylic acid compound via an isocyanate compound.

Although the cyclic carbodiimide compound is capable of effectively capping acidic groups of a polymer compound, if desired, without departing from the gist of the invention, for example, a conventionally known carboxyl-group-capping agent for polymers can be used together. Examples of such conventionally known carboxyl-group-capping agents include agents described in JP-A-2005-2174, such as an epoxy compound, an oxazoline compound, and an oxazine compound.

### <Polymer Compound>

In the invention, a polymer compound to which the cyclic carbodiimide compound is applied has acidic groups. The acidic group may be at least one member selected from the group consisting of a carboxyl group, a sulfonic acid group, a sulfinic acid group, a phosphonic acid group, and a phosphinic acid group. The polymer compound may be at least one member selected from the group consisting of polyesters, polyamides, polyamideimides, polyimides, and polyester amides.

Examples of polyesters include polymers and copolymers obtained by the polycondensation of at least one member selected from a dicarboxylic acid or an ester-forming derivative thereof with a diol or an ester-forming derivative thereof, a hydroxycarboxylic acid or an ester-forming derivative thereof, and a lactone. Thermoplastic polyester resins are preferable, for example. For moldability, etc., such a thermoplastic polyester resin may have a crosslinked structure formed by treatment with a radical-generating source, such as active energy rays or an oxidizing agent.

Examples of dicarboxylic acids and ester-forming derivatives thereof include aromatic dicarboxylic acids such as terephthalic acid, isophthalic acid, phthalic acid, 2,6-naphthalenedicarboxylic acid, 1,5-naphthalenedicarboxylic acid, bis(p-carboxyphenyl)methane, anthracenedicarboxylic acid, 4,4'-diphenyl ether dicarboxylic acid, 5-tetrabutylphosphonium isophthalic acid, and 5-sodium sulfoisophthalic acid, as well as ester-forming derivatives thereof. Examples also include aliphatic dicarboxylic acids such as oxalic acid, succinic acid, adipic acid, sebacic acid, azelaic acid, dodecanedioic acid, malonic acid, glutaric acid, and dimer acid, as well as ester-forming derivatives thereof. Examples also include alicyclic dicarboxylic acids such as 1,3-cyclohexanedicarboxylic acid and 1,4-cyclohexanedicarboxylic acid, as well as ester-forming derivatives thereof.

Examples of diols and ester-forming derivatives thereof include C₂₋₂₀ aliphatic glycols, i.e., ethylene glycol, propylene glycol, 1,3-propanediol, 1,4-butanediol, neopentyl glycol, 1,5-pentanediol, 1,6-hexanediol, decamethylene glycol, cyclohexane dimethanol, cyclohexanediol, dimer diol, and the like. Examples also include long-chain glycols having a molecular weight of 200 to 100,000, i.e., polyethylene glycol, polytrimethylene glycol, poly(1,2-propylene glycol), polytetramethylene glycol, and the like. Examples also include aromatic dioxy compounds, i.e., 4,4'-dihydroxybiphenyl, hydroquinone, tert-butyl hydroquinone, bisphenol-A, bisphenol-S, bisphenol-F, and the like, as well as ester-forming derivatives thereof.

Examples of hydroxycarboxylic acids include glycolic acid, lactic acid, hydroxypropionic acid, hydroxybutyric acid, hydroxyvaleric acid, hydroxycaproic acid, hydroxybenzoic acid, p-hydroxybenzoic acid, and 6-hydroxy-2-naphthoic acid, as well as ester-forming derivatives thereof. Examples of lactones include caprolactone, valerolactone, propiolactone, undecalactone, and 1,5-oxepan-2-one.

Examples of aromatic polyesters obtained by the polycondensation of, as main components, an aromatic dicarboxylic acid or an ester-forming derivative thereof and an aliphatic diol or an ester-forming derivative thereof include polymers obtained by the polycondensation of, as main components, an aromatic carboxylic acid or an ester-forming derivative thereof, preferably terephthalic acid, naphthalene-2,6-dicarboxylic acid, or an ester-forming derivative thereof, and an aliphatic diol selected from ethylene glycol, 1,3-propanediol, and butanediol or an ester-forming derivative thereof.

Specific preferred examples thereof include polyethylene terephthalate, polyethylene naphthalate, polytrimethylene terephthalate, polytrimethylene naphthalate, polybutylene terephthalate, polybutylene naphthalate, polyethylene(terephthalate/isophthalate), polytrimethylene(terephthalate/isophthalate), polybutylene(terephthalate/isophthalate), polyethylene terephthalate-polyethylene glycol, polytrimethylene terephthalate-polyethylene glycol, polybutylene terephthalate-polyethylene glycol, polybutylene naphthalate-polyethylene glycol, polyethylene terephthalate-poly(tetramethylene oxide) glycol, polytrimethylene terephthalate-poly(tetramethylene oxide) glycol, polybutylene terephthalate-poly(tetramethylene oxide) glycol, polybutylene naphthalate-poly(tetramethylene oxide) glycol, polyethylene(terephthalate/isophthalate)-poly(tetramethylene oxide) glycol, polytrimethylene(terephthalate/isophthalate)-poly(tetramethylene oxide) glycol, polybutylene(terephthalate/isophthalate)-poly(tetramethylene oxide) glycol, polybutylene(terephthalate/succinate), polyethylene(terephthalate/succinate), polybutylene(terephthalate/adipate), and polyethylene(terephthalate/adipate).

Examples of aliphatic polyesters include polymers containing an aliphatic hydroxycarboxylic acid as a main component, polymers obtained by the polycondensation of an aliphatic polycarboxylic acid or an ester-forming derivative thereof and an aliphatic polyalcohol as main components, and copolymers thereof.

Examples of polymers containing an aliphatic hydroxycarboxylic acid as a main component inclucde polycondensates of glycolic acid, lactic acid, hydroxypropionic acid, hydroxybutyric acid, hydroxyvaleric acid, hydroxycaproic acid, and the like, as well as copolymers thereof. In particular, polyglycolic acid, polylactic acid, poly(3-hydroxycarboxybutyric acid), poly(4-polyhydroxybutyric acid), poly(3-hydroxyhexanoic acid), polycaprolactone, copolymers thereof, and the like are mentioned. Poly(L-lactic acid), poly(D-lactic acid), stereocomplex polylactic acid, and racemic polylactic acid are particularly suitable.

Examples of polyesters also include polymers containing an aliphatic polycarboxylic acid and an aliphatic polyalcohol as main components. Examples of polycarboxylic acids include aliphatic dicarboxylic acids such as oxalic acid, succinic acid, adipic acid, sebacic acid, azelaic acid, dodecanedioic acid, malonic acid, glutaric acid, and dimer acid. Examples also include alicyclic dicarboxylic acid units such as 1,3-cyclohexanedicarboxylic acid and 1,4-cyclohexanedicarboxylic acid, as well as ester derivatives thereof.

Examples of diol components include C₂₋₂₀ aliphatic glycols, i.e., ethylene glycol, 1,3-propanediol, 1,4-butanediol, neopentyl glycol, 1,5-pentanediol, 1,6-hexanediol, decamethylene glycol, cyclohexane dimethanol, cyclohexanediol, dimer diol, and the like. Examples also include condensates containing as a main component a long-chain glycol having a molecular weight of 200 to 100,000, i.e., polyethylene glycol, polytrimethylene glycol, poly(1,2-propylene glycol), or polytetramethylene glycol. Specific examples thereof include polyethylene adipate, polyethylene succinate, polybutylene adipate, and polybutylene succinate, as well as copolymers thereof.

Further, examples of wholly aromatic polyesters include polymers obtained by the polycondensation of, as main components, an aromatic carboxylic acid or an ester-forming derivative thereof, preferably terephthalic acid, naphthalene-2,6-dicarboxylic acid, or an ester-forming derivative thereof, and an aromatic polyhydroxy compound or an ester-forming derivative thereof.

Specific examples thereof include poly(4-oxyphenylene-2,2-propylidene-4-oxyphenylene-terephthaloyl-co-isophthaloyl).

Such a polyester has, as carbodiimide-reactive components, terminal carboxyl and/or hydroxyl groups at its molecular ends in an amount of 1 to 50 eq/ton. Such terminal groups, especially carboxyl groups, reduce the stability of the polyester and thus are preferably capped with a cyclic carbodiimide compound.

In the capping of terminal carboxyl groups with a carbodiimide compound, the application of the cyclic carbodiimide compound of the invention allows the carboxyl groups to be capped without producing toxic, free isocyanates. This is greatly advantageous.

Further, as an additional effect, because of chain extension by the terminal isocyanate groups that are not released but formed in the polyester during capping with the cyclic carbodiimide compound and the terminal hydroxyl or carboxyl groups that are present in the polyester, the molecular weight of the polyester can be increased or prevented from decreasing more efficiently as compared with conventional linear carbodiimide compounds. This is of great industrial significance.

The polyesters mentioned above can be produced by a well known method (e.g., described in *"*Howa-Poriesuteru-Jushi Handobukku (Handbook of Saturated Polyester Resin)" written by Kazuo YUKI, Nikkan Kogyo Shimbun (published on December 22, 1989), etc.).

In the invention, examples of polyesters further include, in addition to the above polyesters, unsaturated polyester resins obtained by the copolymerization of unsaturated polycarboxylic acids or ester-forming derivatives thereof and also polyester elastomers containing a low-melting-point polymer segment.

Examples of unsaturated polycarboxylic acids include maleic anhydride, tetrahydromaleic anhydride, fumaric acid, and endomethylene tetrahydromaleic anhydride. Various monomers are added to such an unsaturated polyester in order to control curing properties, and the unsaturated polyester is cured and molded by heat curing, radical curing, or curing with active energy rays such as light or electron beams. The control of carboxyl groups in such an unsaturated resin is an important technical problem related to rheological properties such as thixotropy, resin durability, etc. However, the cyclic carbodiimide compound allows the carboxyl groups to be capped and controlled without producing toxic, free isocyanates, and also allows the molecular weight to be more effectively increased. These advantages are of great industrial significance.

Further, in the invention, the polyester may also be a polyester elastomer obtained by the copolymerization of soft components. A polyester elastomer is a copolymer containing a high-melting-point polyester segment and a low-melting-point polymer segment having a molecular weight of 400 to 6,000, as described in known documents, for example, JP-A-11-92636.

In the case where the copolymer is made solely of a high-melting-point polyester segment, the melting point thereof is 150°C or more. In the case where the copolymer is made solely of a low-melting-point polymer segment, the melting point or softening point thereof is 80°C or less. It is preferable that a low-melting-point polymer segment is made of a polyalkylene glycol or a C₂₋₁₂ aliphatic dicarboxylic acid and a C₂₋₁₀ aliphatic glycol. Such an elastomer has a problem with hydrolytic stability. However, its carboxyl groups can be controlled by the cyclic carbodiimide compound without any safety problem, which is of great significance, and also its molecular weight can be prevented from decreasing or can be increased by the cyclic carbodiimide compound, which is of great industrial significance.

As a polyamide, a thermoplastic polymer having an amide bond and containing an amino acid, a lactam, or a diamine and a dicarboxylic acid or an amide-forming derivative thereof as main raw materials is mentioned.

In the invention, polycondensates obtained by the condensation of a diamine and a dicarboxylic acid or an acyl activator thereof, polymers obtained by the polycondensation of an aminocarboxylic acid, a lactam, or an amino acid, and copolymers thereof are usable as polyamides.

Examples of diamines include aliphatic diamines and aromatic diamines. Examples of aliphatic diamines include tetramethylenediamine, hexamethylenediamine, undecamethylenediamine, dodecamethylenediamine, 2,2,4-trimethylhexamethylenediamine, 2,4,4-trimethylhexamethylenediamine, 5-methylnonamethylenediamine, 2,4-dimethyloctamethylenediamine, m-xylylenediamine, p-xylylenediamine, 1,3-bis(aminomethyl)cyclohexane, 1-amino-3-aminomethyl-3,5,5-trimethylcyclohexane, 3,8-bis(aminomethyl)tricyclodecane, bis(4-aminocyclohexyl)methane, bis(3-methyl-4-aminocyclohexyl) methane, 2,2-bis(4-aminocyclohexyl)propane, bis(aminopropyl)piperazine, and aminoethylpiperazine.

Examples of aromatic diamines include p-phenylenediamine, m-phenylenediamine, 2,6-naphthalenediamine, 4,4'-diphenyldiamine, 3,4'-diphenyldiamine, 4,4'-diaminodiphenyl ether, 3,4'-diaminodiphenyl ether, 4,4'-diaminodiphenyl sulfone, 3,4'-diaminodiphenyl sulfone, 4,4'-diaminodiphenyl ketone, 3,4'-diaminodiphenyl ketone, and 2,2-bis(4-aminophenyl)propane.

Examples of dicarboxylic acids include adipic acid, suberic acid, azelaic acid, sebacic acid, dodecanoic acid, terephthalic acid, isophthalic acid, naphthalenedicarboxylic acid, 2-chloroterephthalic acid, 2-methylterephthalic acid, 5-methylisophthalic acid, 5-sodium sulfoisophthalic acid, hexahydroterephthalic acid, hexahydroisophthalic acid, and diglycolic acid.

Specific examples of polyamides include aliphatic polyamides such as polycaproamide (Nylon 6), polytetramethylene adipamide (Nylon 46), polyhexamethylene adipamide (Nylon 66), polyhexamethylene sebacamide (Nylon 610), polyhexamethylene dodecamide (Nylon 612), polyundecamethylene adipamide (Nylon 116), polyundecanamide (Nylon 11), and polydodecanamide (Nylon 12).

Examples also include aliphatic-aromatic polyamides such as polytrimethylhexamethylene terephthalamide, polyhexamethylene isophthalamide (Nylon 6I), polyhexamethylene terephthal/isophthalamide (Nylon 6T/6I), poly[bis(4-aminocyclohexyl)methane dodecamide] (Nylon PACM12), poly[bis(3-methyl-4-aminocyclohexyl)methane dodecamide] (Nylon Dimethyl PACM12), poly(m-xylylene adipamide) (Nylon MXD6), polyundecamethylene terephthalamide (Nylon 11T), polyundecamethylene hexahydroterephthalamide (Nylon 11T(H)), and copolyamides thereof, as well as copolymers and mixtures thereof. Examples further include poly(p-phenylene terephthalamide) and poly(p-phenylene terephthalamide-co-isophthalamide).

Examples of amino acids include ω-aminocaproic acid, ω-aminoenanthic acid, ω-aminocaprylic acid, ω-aminopergonic acid, ω-aminocapric acid, 11-aminoundecanoic acid, 12-aminododecanoic acid, and p-aminomethylbenzoic acid. Examples of lactams include ω-caprolactam, ω-enantholactam, ω-capryllactam, and ω-laurolactam.

The molecular weight of such a polyamide resin is not particularly limited. However, it is preferable that its relative viscosity measured at 25°C in a 98% concentrated sulfuric acid solution having a polyamide resin concentration of 1% by weight is within a range of 2.0 to 4.0.

These amide resins can be produced according to a well known method, for example, *"*poriamido-Jusi Handobukku (Polyamide Resin Handbook)" (written by Osamu FUKUMOTO, Nikkan Kogyo Shimbun, published on January 30, 1988), etc.

Polyamides in the invention further include polyamides known as polyamide elastomers. Examples of such polyamides include graft and block copolymers obtained by a reaction of a polyamide-forming component having 6 or more carbon atoms with a poly(alkylene oxide) glycol. The linkage between the polyamide-forming component having 6 or more carbon atoms and the poly(alkylene oxide) glycol component is usually an ester bond or an amide bond. However, the linkage is not particularly limited thereto, and it is also possible to use a third component, such as a dicarboxylic acid or a diamine, as a reaction component for the two.

Examples of poly(alkylene oxide) glycols include polyethylene oxide glycol, poly(1,2-propylene oxide) glycol, poly(1,3-propylene oxide) glycol, poly(tetramethylene oxide) glycol, poly(hexamethylene oxide) glycol, block and random copolymers of ethylene oxide and propylene oxide, and block and random copolymers of ethylene oxide and tetrahydrofuran. In terms of polymerizability and rigidity, the number average molecular weight of the poly(alkylene oxide) glycol is preferably 200 to 6,000, and more preferably 300 to 4,000. As a polyamide elastomer for use in the invention, a polyamide elastomer obtained by the polymerization of caprolactam, polyethylene glycol, and terephthalic acid is preferable.

As can be easily understood from the raw materials, such a polyamide resin has carboxyl groups in an amount of 30 to 100 eq/ton and amino groups in an amount of 30 to 100 eq/ton, approximately, but it is well known that carboxyl groups have an unfavorable effect on the stability of a polyamide.

By the cyclic carbodiimide compound of the invention, the carboxyl groups are controlled to 20 eq/ton or less or to 10 eq/ton or less, preferably further to a lower degree, without any safety problems, and also the molecular weight is more effectively prevented from decreasing; such a composition is of great significance.

A polyamideimide resin for use in the invention has a main repeating structural unit represented by the following formula (I).

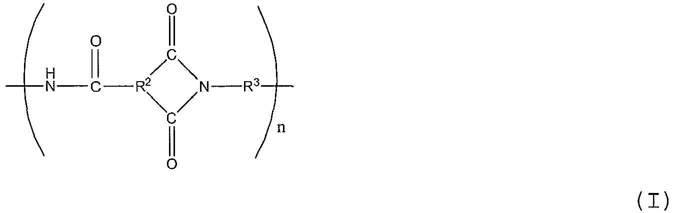

In the formula, R² represents a trivalent organic group, R³ represents a divalent organic group, and n represents a positive integer.

Examples of typical methods for synthesizing such a polyamideimide resin include (1) a method in which a diisocyanate is allowed to react with a tribasic acid anhydride, (2) a method in which a diamine is allowed to react with a tribasic acid anhydride, and (3) a method in which a diamine is allowed to react with a tribasic acid anhydride chloride. However, the method for synthesizing a polyamideimide resin for use in the invention is not limited thereto. Typical compounds used in the above synthesizing methods are listed hereinafter.

First, preferred examples of diisocyanates include 4,4'-diphenylmethane diisocyanate, xylylene diisocyanate, 3,3'-diphenylmethane diisocyanate, 4,4'-diphenylether diisocyanate, 3,3'-diphenylether diisocyanate, and p-phenylene diisocyanate.

Preferred examples of diamines include 4,4'-diaminodiphenyl sulfone, 3,3'-diaminodiphenyl sulfone, 4,4'-diaminodiphenyl ether, 3,3'-diaminodiphenyl ether, 4,4'-diaminodiphenylmethane, 3,3'-diaminodiphenylmethane, xylylenediamine, and phenylenediamine.

Among these, 4,4'-diphenylmethane diisocyanate, 3,3'-diphenylmethane diisocyanate, 4,4'-diphenylether diisocyanate, 3,3'-diphenylether diisocyanate, 4,4'-diaminodiphenyl ether, 3,3'-diaminodiphenyl ether, 4,4'-diaminodiphenylmethane, and 3,3'-diaminodiphenylmethane are more preferable.

Preferred examples of tribasic acid anhydrides include trimellitic anhydride, and examples of tribasic acid anhydride chlorides include trimellitic anhydride chloride.

In the synthesis of a polyamideimide resin, a dicarboxylic acid, a tetracarboxylic dianhydride, or the like may be simultaneously subjected to the reaction without impairing the properties of the polyamideimide resin. Examples of dicarboxylic acids include terephthalic acid, isophthalic acid, and adipic acid. Examples of tetracarboxylic dianhydrides include pyromellitic dianhydride, benzophenone tetracarboxylic dianhydride, and biphenyl tetracarboxylic dianhydride. It is preferable that they are used in an amount of 50 eq% or less based on the total acid components.

The durability of a polyamideimide resin may decrease depending on the concentration of carboxyl groups contained in the polymer. Therefore, it is preferable that the carboxyl group content is controlled preferably to 1 to 10 eq/ton or less. In the cyclic carbodiimide compound of the invention, the above carboxyl group concentration range can be suitably achieved.

As a polyimide resin, it is preferable to select a thermoplastic polyimide. An example of a polyimide resin is a polyimide containing the following diamine component and tetracarboxylic acid:

H₂N-R⁴-NH₂

wherein R⁴ is (i) a single bond; (ii) a C₂₋₁₂ aliphatic hydrocarbon group; (iii) a C₄₋₃₀ alicyclic group; (iv) a C₆-30 aromatic group; (v) a -Ph-O-R⁵-O-Ph- group (in the formula, R₅ represents a phenylene group or a Ph-W¹-Ph-group wherein W¹ represents a single bond, a C₁₋₄ alkylene group optionally substituted with a halogen atom, a -O-Ph-O- group, -O-, -CO-, -S-, -SO-, or a -SO₂ group; or (vi) a -R⁶-(SiR⁷₂O)ₘ-SiR⁷₂-R⁶- group (in the formula, R⁶ represents (CH₂)ₛ-, -(CH₂)ₛ-Ph-, -(CH₂)ₛ-O-Ph-, or -Ph-wherein m is an integer of 1 to 100, s represents an integer of 1 to 4, and R⁷ represents a C₁₋₆ alkyl group, a phenyl group, or a C₁₋₆ alkylphenyl group), 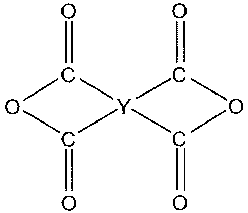 wherein Y is a C₂₋₁₂ tetravalent aliphatic group, a C₄₋₈ tetravalent alicyclic group, a C₆₋₁₄ monocyclic or fused-ring polycyclic tetravalent aromatic group, or a >Ph-W²-Ph< group (in the formula, W² represents a single bond, a C₁₋₄ alkylene group optionally substituted with a halogen atom, -O-Ph-O-, -O-, -CO-, -S-, -SO-, or a -SO₂- group).

Specific examples of tetracarboxylic anhydrides for use in the production of a polyamide acid include, but are not limited to, pyromellitic anhydride (PMDA), 4,4'-oxydiphthalic anhydride (ODPA), biphenyl-3,3',4,4'-tetracarboxylic anhydride (BPDA), benzophenone-3,3',4,4'-tetracarboxylic anhydride (BTDA), ethylenetetracarboxylic anhydride, butanetetracarboxylic anhydride, cyclopentanetetracarboxylic anhydride, benzophenone-2,2',3,3'-tetracarboxylic anhydride, biphenyl-2,2',3,3'-tetracarboxylic anhydride, 2,2-bis(3,4-dicarboxyphenyl)propane anhydride, 2,2-bis(2,3-dicarboxyphenyl)propane anhydride, bis(3,4-dicarboxyphenyl)ether anhydride, bis(3,4-dicarboxyphenyl)sulfone anhydride, 1,1-bis(2,3-dicarboxyphenyl)ethane anhydride, bis(2,3-dicarboxyphenyl)methane anhydride, bis(3,4-dicarboxyphenyl)methane anhydride, 4,4'-(p-phenylenedioxy)diphthalic anhydride, 4,4'-(m-phenylenedioxy)diphthalic anhydride, naphthalene-2,3,6,7-tetracarboxylic anhydride, naphthalene-1,4,5,8-tetracarboxylic anhydride, naphthalene-1,2,5,6-tetracarboxylic anhydride, benzene-1,2,3,4-tetracarboxylic anhydride, perylene-3,4,9,10-tetracarboxylic anhydride, anthracene-2,3,6,7-tetracarboxylic anhydride, and phenanthrene-1,2,7,8-tetracarboxylic anhydride.

These dicarboxylic anhydrides may be used alone, and it is also possible to use a mixture of two or more kinds. Among them, it is preferable to use pyromellitic anhydride (PMDA), 4,4'-oxydiphthalic anhydride (ODPA), biphenyl-3,3',4,4'-tetracarboxylic anhydride (BPDA), benzophenone-3,3',4,4'-tetracarboxylic anhydride, or biphenylsulfone-3,3',4,4'-tetracarboxylic anhydride (DSDA).

Specific example of diamines for use in the production of a polyimide include, but are not limited to, 4,4'-diaminodiphenyl ether, 4,4'-diaminodiphenylmethane, 4,4'-diaminodiphenyl sulfone, 4,4'-diaminodiphenyl thioether, 4,4'-di(m-aminophenoxy)diphenyl sulfone, 4,4'-di(p-aminophenoxy)diphenyl sulfone, o-phenylenediamine, m-phenylenediamine, p-phenylenediamine, benzidine, 2,2'-diaminobenzophenone, 4,4'-diaminobenzophenone, 4,4'-diaminodiphenyl-2,2'-propane, 1,5-diaminonaphthalene, 1,8-diaminonaphthalene, trimethylenediamine, tetramethylenediamine, hexamethylenediamine, 4,4-dimethylheptamethylenediamine, 2,11-dodecadiamine, di(p-aminophenoxy)dimethylsilane, 1,4-di(3-aminopropyldiaminosilane)benzene, 1,4-diaminocyclohexane, o-tolyldiamine, m-tolyldiamine, acetoguanamine, benzoguanamine, 1,3-bis(3-aminophenoxy)benzene (APB), bis[4-(3-aminophenoxy)phenyl]methane, 1,1-bis[4-(3-aminophenoxy)phenyl] ethane, 1,2-bis[4- (3-aminophenoxy)phenyl] ethane, 2,2-bis[4-(3-aminophenoxy)phenyl]ethane, 2,2-bis[4-(3-aminophenoxy)phenyl]propane, 2,2-bis[4-(3-aminophenoxy)phenyl]butane, 2,2-bis[4-(3-aminophenoxy)phenyl]-1,1,1,3,3,3-hexafluoropropane, 4,4'-di(3-aminophenoxy)biphenyl, di[4-(3-aminophenoxy)phenyl]ketone, di[4-(3-aminophenoxy)phenyl]sulfide, di[4-(3-aminophenoxy)phenyl]sulfoxide, di[4-(3-aminophenoxy)phenyl]sulfone, and di(4-(3-amionohpenoxy)phenyl)ether. The above diamines may be used alone, and it is also possible to use a mixture of a large number of them.

Examples of thermoplastic polyimides include polyimide resins containing a tetracarboxylic anhydride represented by the below formula and a known diamine such as p-phenylenediamine, cyclohexanediamine, or hydrogenated bisphenol-A type diamine. Further, examples also include those commercially available from General Electric under the trade name Ultem, such as Ultem 1000, Ultem 1010, Ultem CRS5001, and Ultem XH6050, and also AURUM 250AM manufactured by Mitsui Chemicals.

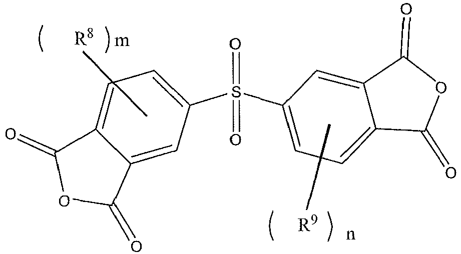

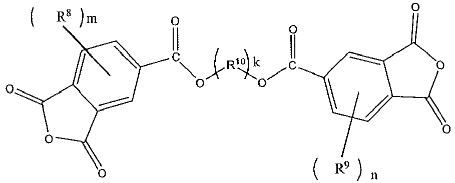

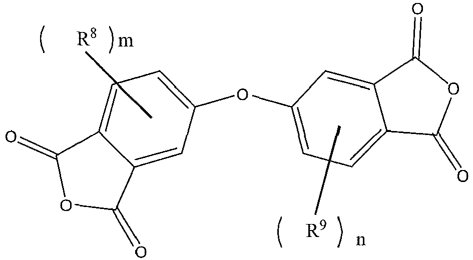

In the formulae, R⁸ and R⁹ each independently represent a hydrogen atom, a linear or branched C₁₋₁₀ alkyl group, or an aryl group, R¹⁰ represents a C₆₋₃₀ arylene group or a C₂₋₂₀ alkylene group, m and n are each an integer of 0 to 5, and k is an integer of 1 to 3.

Examples of polyester amide resins include conventionally known polyester amide resins obtained by the copolymerization of a polyester component and a polyamide component. In particular, it is preferable to select a thermoplastic polyester amide resin.

A polyester amide resin can be synthesized by a known method, etc. For example, it is possible to employ a method in which the polyamide component is first subjected to a polycondensation reaction to synthesize a polyamide terminated with functional groups, and then the polyester component is polymerized in the presence of the polyamide, etc. This polycondensation reaction usually takes place through the first stage in which an amidation reaction proceeds and then the second stage in which an esterification reaction proceeds. The polyester component is preferably selected from the polyester components mentioned above. In addition, the polyamide component is preferably selected from the polyamide components mentioned above.

### <Use of Cyclic Carbodiimide Compound>

In the invention, the cyclic carbodiimide compound is mixed with a polymer compound having acidic groups to cause a reaction therebetween, whereby the acidic groups can be capped. The method for mixing the cyclic carbodiimide compound into the polymer compound is not particularly limited and may be a conventionally known method. It is possible to employ a method in which the cyclic carbodiimide compound is added in the form of a solution, a melt, or a masterbatch of a polymer to be treated, a method in which a polymer compound in solid state is brought into contact with a liquid having the cyclic carbodiimide compound dissolved, dispersed, or melted therein, thereby impregnating the polymer compound with the cyclic carbodiimide compound, or the like.

In the case where a method in which the cyclic carbodiimide compound is added in the form of a solution, a melt, or a masterbatch of a polymer to be treated is employed, it is possible to employ a method in which a conventionally known kneading apparatus is used for addition. For kneading, kneading in the form of a solution or kneading in the form of a melt is preferable in terms of uniform kneading. The kneading apparatus is not particularly limited and may be a conventionally known vertical reaction vessel, mixing tank, kneading tank, or a single-screw or multi-screw horizontal kneading apparatus such as a single-screw or multi-screw extruder or kneader, for example. The mixing time with a polymer compound is not particularly limited. Although this depends on the mixing apparatus and the mixing temperature, the mixing time is preferably 0.1 minutes to 2 hours, more preferably 0.2 minutes to 60 minutes, and still more preferably 0.2 minutes to 30 minutes.

The solvent may be one that is inert to the polymer compound and the cyclic carbodiimide compound. In particular, a solvent that has affinity for both of them and at least partially dissolves both of them is preferable.

Examples of usable solvents include hydrocarbon-based solvents, ketone-based solvents, ester-based solvents, ether-based solvents, halogen-based solvents, and amide-based solvents.

Examples of hydrocarbon-based solvents include hexane, cyclohexane, benzene, toluene, xylene, heptane, and decane. Examples of ketone-based solvents include acetone, methyl ethyl ketone, diethyl ketone, cyclohexanone, and isophorone. Examples of ester-based solvents include ethyl acetate, methyl acetate, ethyl succinate, methyl carbonate, ethyl benzoate, and diethylene glycol diacetate. Examples of ether-based solvents include diethyl ether, dibutyl ether, tetrahydrofuran, dioxane, diethylene glycol dimethyl ether, triethylene glycol diethyl ether, and diphenyl ether.

Examples of halogen-based solvents include dichloromethane, chloroform, tetrachloromethane, dichloroethane, 1,1',2,2'-tetrachloroethane, chlorobenzene, and dichlorobenzene. Examples of amide-based solvents include formamide, N,N-dimethylformamide, N,N-dimethylacetamide, and N-methyl-2-pyrrolidone. These solvents may be used alone. They may also be used as a mixed solvent if desired.

In the invention, the solvent is used in an amount within a range of 1 to 1,000 parts by weight per 100 parts by weight of the total of the polymer compound and the cyclic carbodiimide compound. When the amount is less than 1 part by weight, the application of the solvent has no significance. Although there is no particular upper limit on the amount of the solvent to be used, in terms of operativity and reaction efficiency, the upper limit is about 1,000 parts by weight.

In the case where a method in which a polymer compound in solid state is brought into contact with a liquid having the cyclic carbodiimide compound dissolved, dispersed, or melted therein, thereby impregnating the polymer compound with the cyclic carbodiimide compound, is employed, it is possible to employ a method in which the polymer compound in solid state is brought into contact with the carbodiimide compound dissolved in the solvent, a method in which the polymer compound in solid state is brought into contact with an emulsion of the cyclic carbodiimide compound, or the like. As a contact method, it is preferable to employ a method in which the polymer compound is immersed in the cyclic carbodiimide compound, a method in which the cyclic carbodiimide compound is applied or sprayed to the polymer compound, or the like.

The capping reaction of the cyclic carbodiimide compound of the invention can take place at a temperature of room temperature (25°C) to 300°C, approximately. However, in terms of reaction efficiency, the temperature is preferably within a range of 50 to 280°C, more preferably 100 to 280°C, whereby the reaction is further promoted. The reaction easily proceeds at a temperature where the polymer compound is molten. However, in order to prevent the cyclic carbodiimide compound from sublimation, decomposition, or the like, it is preferable to carry out the reaction at a temperature of less than 300°C. The application of a solvent is also effective in reducing the polymer melting temperature and increasing stirring efficiency.

Although the reaction proceeds rapidly enough in the absence of a catalyst, it is also possible to use a catalyst to promote the reaction. As the catalyst, catalysts used for conventional linear carbodiimide compounds (JP-A-2005-2174) are applicable. Examples thereof include alkali metal compounds, alkaline-earth metal compounds, tertiary amine compounds, imidazole compounds, quaternary ammonium salts, phosphine compounds, phosphonium salts, phosphoric acid esters, organic acids, and Lewis acid. They may be used alone, and it is also possible to use two or more kinds. The amount of the catalyst to be added is not particularly limited, but is preferably 0.001 to 1 part by weight, more preferably 0.01 to 0.1 parts by weight, and most preferably 0.02 to 0.1 parts by weight per 100 parts by weight of the total of the polymer compound and the cyclic carbodiimide compound.

The amount of the cyclic carbodiimide compound to be applied is selected such that the amount of carbodiimide groups contained in the cyclic carbodiimide compound is within a range of 0.5 to 100 equivalents per equivalent of acidic groups. When the amount is less than 0.5 equivalents, the application of the carbodiimide may have no significance. When the amount is more than 100 equivalents, the properties of the substrate may change. From such a point of view, based on the above basis, the amount is preferably selected within a range of 0.6 to 75 equivalents, more preferably 0.65 to 50 equivalents, still more preferably 0.7 to 30 equivalents, and particularly preferably 0.7 to 20 equivalents.

### Examples

Hereinafter, the invention will be described in further detail through examples. Physical properties were measured by the following methods.

### (1) Identification of Cyclic Carbodiimide Structure by NMR:

A synthesized cyclic carbodiimide compound was confirmed by ¹H-NMR and ¹³C-NMR. JNR-EX270 manufactured by JEOL was used for NMR. Deuterated chloroform was used as the solvent.

### (2) Identification of Carbodiimide Backbone of Cyclic Carbodiimide by IR:

The presence of the carbodiimide backbone of a synthesized cyclic carbodiimide compound was confirmed by FT-IR at 2,100 to 2,200 cm⁻¹, which is characteristic to a carbodiimide. Magna-750 manufactured by Thermo Nicolet was used for FT-IR.

### (3) Carboxyl Group Concentration

A sample was dissolved in purified o-cresol, dissolved in a nitrogen stream, and titrated with an ethanol solution of 0.05 N potassium hydroxide using bromocresol blue as an indicator.

### Example 1: Synthesis of Cyclic Carbodiimide CC1 (Scheme 1) CC1: MW = 312

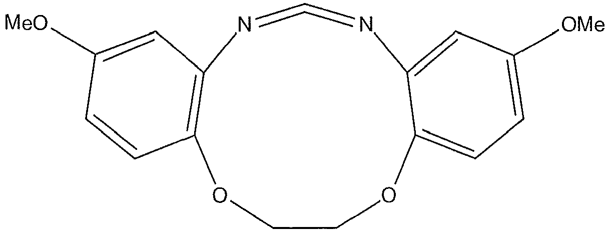

### Step (1a)

4-Methoxy-2-nitrophenol (0.11 mol), 1,2-dibromoethane (0.05 mol), potassium carbonate (0.33 mol), and 200 ml of N,N-dimethylformamide were placed in a reactor equipped with a stirrer and a heater in a N₂ atmosphere and allowed to react at 130°C for 12 hours. DMF was then removed under reduced pressure. The resulting solid was dissolved in 200 ml of dichloromethane, followed by partitioning with 100 ml of water three times. The organic layer was dried over 5 g of sodium sulfate, and dichloromethane was removed under reduced pressure to give an intermediate product A (nitro compound).

### Step (2a)

Next, the intermediate product A (0.1 mol), 5% palladium carbon (Pd/C) (1 g), and 200 ml of ethanol/dichloromethane (70/30) were placed in a reactor equipped with a stirrer. The reactor was purged with hydrogen five times, and the mixture was allowed to react at 25°C under constant supply of hydrogen. The reaction is terminated when hydrogen stops decreasing. Pd/C was recovered, and the mixed solvent was removed to give an intermediate product B (amine compound).

### Step (3a)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product B (0.05 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product C (triphenylphosphine compound).

### Step (4a)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product C (0.05 mol) is slowly added dropwise at 25°C. After dropping, the mixture is allowed to react for 12 hours. Subsequently, dichloromethane was removed, and the resulting solid was purified to give CC1. The structure of CC1 was confirmed by NMR and IR.

### Example 2: Synthesis of Cyclic Carbodiimide CC2 (Scheme 1) CC2: MW = 636

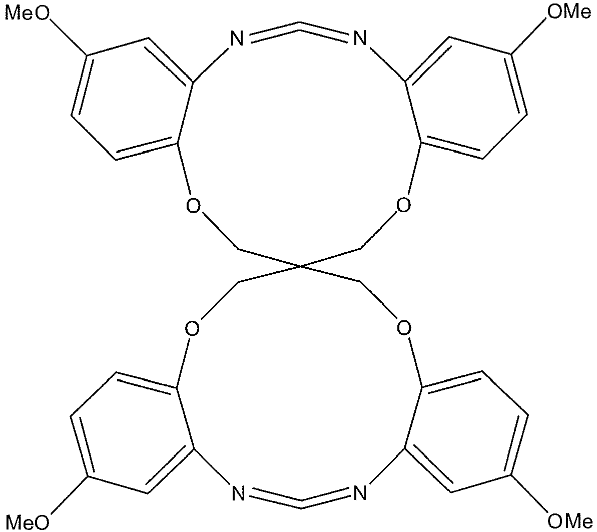

### Step (1A)

4-Methoxy-2-nitrophenol (0.11 mol), pentaerythrityl tetrabromide (0.025 mol), potassium carbonate (0.33 mol), and 200 ml of N,N-dimethylformamide were placed in a reactor equipped with a stirrer and a heater in a N₂ atmosphere and allowed to react at 130°C for 12 hours. DMF was then removed under reduced pressure. The resulting solid was dissolved in 200 ml of dichloromethane, followed by partitioning with 100 ml of water three times. The organic layer was dried over 5 g of sodium sulfate, and dichloromethane was removed under reduced pressure to give an intermediate product D (nitro compound).

### Step (2A)

Next, the intermediate product D (0.1 mol), 5% palladium carbon (Pd/C) (2 g), and 400 ml of ethanol/dichloromethane (70/30) were placed in a reactor equipped with a stirrer. The reactor was purged with hydrogen five times, and the mixture was allowed to react at 25°C under constant supply of hydrogen. The reaction is terminated when hydrogen stops decreasing. Pd/C was recovered, and the mixed solvent was removed to give an intermediate product E (amine compound).

### Step (3A)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product E (0.025 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product F (triphenylphosphine compound).

### Step (4A)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product F (0.025 mol) is slowly added dropwise at 25°C. After dropping, the mixture is allowed to react for 12 hours. Subsequently, dichloromethane was removed, and the resulting solid was purified to give CC2. The structure of CC2 was confirmed by NMR and IR.

### Example 3: Synthesis of Cyclic Carbodiimide CC2 (Scheme 2) Step (1A)

4-Methoxy-2-nitrophenol (0.11 mol), pentaerythrityl tetrabromide (0.025 mol), potassium carbonate (0.33 mol), and 200 ml of N,N-dimethylformamide were placed in a reactor equipped with a stirrer and a heater in a N₂ atmosphere and allowed to react at 130°C for 12 hours. N,N-dimethylformamide was then removed under reduced pressure. The resulting solid was dissolved in 200 ml of dichloromethane, followed by partitioning with 100 ml of water three times.

The organic layer was dried over 5 g of sodium sulfate, and dichloromethane was removed under reduced pressure to give an intermediate product D (nitro compound).

### Step (2A)

Next, the intermediate product D (0.1 mol), 5% palladium carbon (Pd/C) (1.25 g), and 500 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 3 L of water to precipitate a solid. The solid was recovered and dried to give an intermediate product E (amine compound).

### Step (3B)

Next, the intermediate product E (0.025 mol), imidazole (0.2 mol), carbon disulfide (0.2 mol), and 150 ml of 2-butanone are placed in a reactor equipped with a stirrer, a heater, and a gas washing bottle containing alkaline water in a N₂ atmosphere. The reaction solution is heated to a temperature of 80°C and allowed to react for 15 hours. After the reaction, the precipitated solid was recovered by filtration and washed to give an intermediate product G (thiourea compound).

### Step (4B)

Next, the intermediate product G (0.025 mol), p-toluenesulfonyl chloride (0.1 mol), and 50 ml of pyridine are placed in a reactor equipped with a stirrer in a N₂ atmosphere and stirred. The mixture is allowed to react at 25°C for 3 hours, and then 150 ml of methanol is added and further stirred at 25°C for 1 hour. The precipitated solid was recovered by filtration and washed to give CC2. The structure of CC2 was confirmed by NMR and IR.

### Example 4: Synthesis of Cyclic Carbodiimide CC2 (Scheme 2) Step (1A)

4-Methoxy-2-nitrophenol (0.11 mol), pentaerythrityl tetrabromide (0.025 mol), potassium carbonate (0.33 mol), and 200 ml of N,N-dimethylformamide were placed in a reactor equipped with a stirrer and a heater in a N₂ atmosphere and allowed to react at 130°C for 12 hours. N,N-dimethylformamide was then removed under reduced pressure. The resulting solid was dissolved in 200 ml of dichloromethane, followed by partitioning with 100 ml of water three times.

The organic layer was dried over 5 g of sodium sulfate, and dichloromethane was removed under reduced pressure to give an intermediate product D (nitro compound).

### Step (2A)

Next, the intermediate product D (0.1 mol), 5% palladium carbon (Pd/C) (1.25 g), and 500 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 3 L of water to precipitate a solid. The solid was recovered and dried to give an intermediate product E (amine compound).

### Step (3B)

Next, the intermediate product E (0.025 mol), imidazole (0.2 mol), and 125 ml of acetonitrile were placed in a reactor equipped with a stirrer, a heater, and a dropping funnel in a N₂ atmosphere, and diphenyl phosphite (0.1 mol) was placed in the dropping funnel. After purging with carbon dioxide five times, diphenyl phosphite is slowly added dropwise with stirring at 25°C under constant supply of carbon dioxide to allow the mixture to react for 15 hours. After the reaction, the precipitated solid was recovered by filtration and washed to give an intermediate product H (urea compound).

### Step (4B)

Next, the intermediate product H (0.025 mol), p-toluenesulfonyl chloride (0.1 mol), and 50 ml of pyridine are placed in a reactor equipped with a stirrer in a N₂ atmosphere and stirred. The mixture is allowed to react at 25°C for 3 hours, and then 150 ml of methanol is added and further stirred at 25°C for 1 hour. The precipitated solid was recovered by filtration and washed to give CC2. The structure of CC2 was confirmed by NMR and IR.

### Example 5: Synthesis of Cyclic Carbodiimide CC2 (Scheme 3)

### Step (1B)

4-Chloro-3-nitroanisole (0.125 mol), pentaerythritol (0.025 mol), potassium carbonate (0.25 mol), tetrabutylammonium bromide (0.018 mol), and 50 ml of N,N-dimethylformamide were placed in a reactor equipped with a stirrer and a heater in a N₂ atmosphere and allowed to react at 130°C for 12 hours. After the reaction, the solution was added to 200 ml of water, and the precipitated solid was recovered by filtration. The solid was washed and dried to give an intermediate product D (nitro compound).

### Step (2A)

Next, the intermediate product D (0.1 mol), 5% palladium carbon (Pd/C) (1.25 g), and 500 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 3 L of water to precipitate a solid. The solid was recovered and dried to give an intermediate product E (amine compound).

### Step (3B)

Next, the intermediate product E (0.025 mol), imidazole (0.2 mol), and 125 ml of acetonitrile were placed in a reactor equipped with a stirrer, a heater, and a dropping funnel in a N₂ atmosphere, and diphenyl phosphite (0.1 mol) was placed in the dropping funnel. After purging with carbon dioxide five times, diphenyl phosphite is slowly added dropwise with stirring at 25°C under constant supply of carbon dioxide to allow the mixture to react for 15 hours. After the reaction, the precipitated solid was recovered by filtration and washed to give an intermediate product H (urea compound).

### Step (4B)

Next, the intermediate product H (0.025 mol), p-toluenesulfonyl chloride (0.1 mol), and 50 ml of pyridine are placed in a reactor equipped with a stirrer in a N₂ atmosphere and stirred. The mixture is allowed to react at 25°C for 3 hours, and then 150 ml of methanol is added and further stirred at 25°C for 1 hour. The precipitated solid was recovered by filtration and washed to give CC2. The structure of CC2 was confirmed by NMR and IR.

### Example 6: Synthesis of Cyclic Carbodiimide CC2 (Scheme 3) Step (1B)

4-Chloro-3-nitroanisole (0.125 mol), pentaerythritol (0.025 mol), potassium carbonate (0.25 mol), tetrabutylammonium bromide (0.018 mol), and 50 ml of N,N-dimethylformamide were placed in a reactor equipped with a stirrer and a heater in a N₂ atmosphere and allowed to react at 130°C for 12 hours. After the reaction, the solution was added to 200 ml of water, and the precipitated solid was recovered by filtration. The solid was washed and dried to give an intermediate product D (nitro compound).

### Step (2A)

Next, the intermediate product D (0.1 mol), 5% palladium carbon (Pd/C) (1.25 g), and 500 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 3 L of water to precipitate a solid. The solid was recovered and dried to give an intermediate product E (amine compound).

### Step (3B)

Next, the intermediate product E (0.025 mol), imidazole (0.2 mol), carbon disulfide (0.2 mol), and 150 ml of 2-butanone are placed in a reactor equipped with a stirrer, a heater, and a gas washing bottle containing alkaline water in a N₂ atmosphere. The reaction solution is heated to a temperature of 80°C and allowed to react for 15 hours. After the reaction, the precipitated solid was recovered by filtration and washed to give an intermediate product G (thiourea compound).

### Step (4B)

Next, the intermediate product G (0.025 mol), p-toluenesulfonyl chloride (0.1 mol), and 50 ml of pyridine are placed in a reactor equipped with a stirrer in a N₂ atmosphere and stirred. The mixture is allowed to react at 25°C for 3 hours, and then 150 ml of methanol is added and further stirred at 25°C for 1 hour. The precipitated solid was recovered by filtration and washed to give CC2. The structure of CC2 was confirmed by NMR and IR.

### Example 7: Synthesis of Cyclic Carbodiimide CC2 (Scheme 4) Step (1B)

4-Chloro-3-nitroanisole (0.125 mol), pentaerythritol (0.025 mol), potassium carbonate (0.25 mol), tetrabutylammonium bromide (0.018 mol), and 50 ml of N,N-dimethylformamide were placed in a reactor equipped with a stirrer and a heater in a N₂ atmosphere and allowed to react at 130°C for 12 hours. After the reaction, the solution was added to 200 ml of water, and the precipitated solid was recovered by filtration. The solid was washed and dried to give an intermediate product D (nitro compound).

### Step (2A)

Next, the intermediate product D (0.1 mol), 5% palladium carbon (Pd/C) (1.25 g), and 500 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 3 L of water to precipitate a solid. The solid was recovered and dried to give an intermediate product E (amine compound).

### Step (3A)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product E (0.025 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product F (triphenylphosphine compound).

### Step (4A)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product F (0.025 mol) is slowly added dropwise at 25°C. After dropping, the mixture is allowed to react for 12 hours. Subsequently, dichloromethane was removed, and the resulting solid was purified to give CC2. The structure of CC2 was confirmed by NMR and IR.

### Example 8: End-Capping of Polylactic Acid with CC1

0.005 parts by weight of tin octylate was added to 100 parts by weight of L-lactide (manufactured by Musashino Chemical Laboratory, optical purity: 100%), and the mixture was allowed to react in a nitrogen atmosphere in a reactor equipped with a stirring blade at 180°C for 2 hours. As a catalyst deactivator, phosphoric acid was added in an amount of 1.2 equivalents of tin octylate, then the residual lactide was removed at 13.3 Pa, and the resulting product was formed into chips to give poly(L-lactic acid). The obtained poly(L-lactic acid) had a carboxyl group concentration of 14 eq/ton.

100 parts by weight of the obtained poly(L-lactic acid) and 0.5 parts by weight of CC1 were melt-kneaded in a twin-screw extruder (cylinder temperature: 230°C) for a residence time of 3 minutes. The carboxyl group concentration had decreased to 0.4 eq/ton or less. In addition, no isocyanate odor was detected at the outlet of the extruder after kneading.

### Example 9: End-Capping of Polylactic Acid with CC2

A reaction was carried out under the same conditions as in Example 8, except that the cyclic carbodiimide CC1 was replaced with the cyclic carbodiimide CC2. As a result, the carboxyl group concentration decreased to 0.3 eq/ton or less. In addition, no isocyanate odor was detected at the outlet of the extruder after kneading.

### Comparative Example 1: End-Capping of Polylactic Acid with Linear Carbodiimide Compound

A reaction was carried out under the same conditions as in Example 8, except that the cyclic carbodiimide compound CC1 was replaced with a linear carbodiimide "Stabaxol" I manufactured by Rhein Chemie Japan. As a result, although the carboxyl group concentration was 0.4 eq/ton, a strong, offensive isocyanate odor was generated at the outlet of the extruder.

### Example 10: End-Capping of Polyamide with CC2

Poly(m-xylene adipamide) ("MX Nylon S6001" manufactured by Mitsubishi Gas Chemical), a polyamide made of m-xylylenediamine and adipic acid and having a carboxyl group concentration of 70 eq/ton, was used. 100 parts by weight of this poly(m-xylene adipamide) and 2.0 parts by weight of CC2 were melt-kneaded in a twin-screw extruder (cylinder temperature: 260°C) for a residence time of 3 minutes. The carboxyl group concentration had decreased to 1.2 eq/ton or less. In addition, no isocyanate odor was detected at the outlet of the extruder after kneading.

### Comparative Example 2: End-capping of Polyamide with Linear Carbodiimide Compound

A reaction was carried out under the same conditions as in Example 10, except that the cyclic carbodiimide compound CC2 was replaced with a linear carbodiimide "Stabaxol" I manufactured by Rhein Chemie Japan. As a result, although the carboxyl group concentration was 2.2 eq/ton, a strong, offensive isocyanate odor was generated at the outlet of the extruder.

### Example 12: Synthesis of Cyclic Carbodiimide CC3 (Scheme 1)

CC3: MW = 328

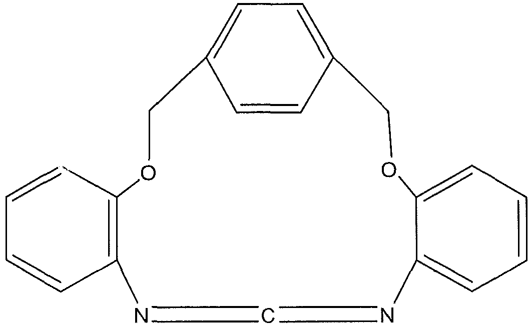

### Step (1a)

o-Nitrophenol (0.11 mol), 1,4-bis(bromomethyl)benzene (0.05 mol), potassium carbonate (0.33 mol), and 200 ml of N,N-dimethylformamide were placed in a reactor equipped with a stirrer and a heater in a N₂ atmosphere and allowed to react at 130°C for 12 hours. DMF was then removed under reduced pressure. The resulting solid was dissolved in 200 ml of dichloromethane, followed by partitioning with 100 ml of water three times. The organic layer was dried over 5 g of sodium sulfate, and dichloromethane was removed under reduced pressure to give an intermediate product I (nitro compound).

### Step (2a)

Next, the intermediate product I (0.1 mol), 5% palladium carbon (Pd/C) (1.5 g), and 300 ml of ethanol/dichloromethane (70/30) were placed in a reactor equipped with a stirrer. The reactor was purged with hydrogen five times, and the mixture was allowed to react at 25°C under constant supply of hydrogen. The reaction is terminated when hydrogen stops decreasing. Pd/C was recovered, and the mixed solvent was removed to give an intermediate product J (amine compound).

### Step (3a)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product J (0.05 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product K (triphenylphosphine compound).

### Step (4a)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product K (0.05 mol) is slowly added dropwise at 25°C. After dropping, the mixture is allowed to react for 12 hours. Subsequently, dichloromethane was removed, and the resulting solid was purified to give CC3. The structure of CC3 was confirmed by NMR and IR.

### Example 13: Synthesis of Cyclic Carbodiimide CC3 (Scheme 2)

### Step (1a)

o-Nitrophenol (0.11 mol), 1,4-bis(bromomethyl)benzene, 1,4-bis(bromomethyl)benzene (0.05 mol), potassium carbonate (0.33 mol), and 200 ml of N,N-dimethylformamide were placed in a reactor equipped with a stirrer and a heater in a N₂ atmosphere and allowed to react at 130°C for 12 hours. DMF was then removed under reduced pressure. The resulting solid was dissolved in 200 ml of dichloromethane, followed by partitioning with 100 ml of water three times. The organic layer was dried over 5 g of sodium sulfate, and dichloromethane was removed under reduced pressure to give an intermediate product I (nitro compound).

### Step (2a)

Next, the intermediate product I (0.1 mol), 5% palladium carbon (Pd/C) (1.5 g), and 200 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 600 ml of water to precipitate a solid. The solid was recovered and dried to give an intermediate product J (amine compound).

### Step (3b)

Next, the intermediate product J (0.025 mol), imidazole (0.1 mol), carbon disulfide (0.1 mol), and 100 ml of 2-butanone are placed in a reactor equipped with a stirrer, a heater, and a gas washing bottle containing alkaline water in a N₂ atmosphere. The reaction solution is heated to a temperature of 80°C and allowed to react for 15 hours. After the reaction, the precipitated solid was recovered by filtration and washed to give an intermediate product L (thiourea compound).

### Step (4b)

Next, the intermediate product L (0.025 mol), p-toluenesulfonyl chloride (0.05 mol), and 40 ml of pyridine are placed in a reactor equipped with a stirrer in a N₂ atmosphere and stirred. The mixture is allowed to react at 25°C for 3 hours, and then 120 ml of methanol is added and further stirred at 25°C for 1 hour. The precipitated solid was recovered by filtration and washed to give CC3. The structure of 3 was confirmed by NMR and IR.

### Example 14: Synthesis of Cyclic Carbodiimide CC4 (Scheme 1)

CC4: Mw = 388

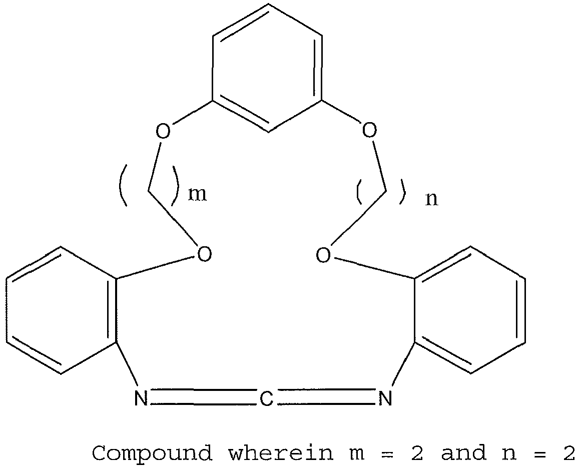

### Step (1b)

o-Chloronitrobenzene (0.0625 mol), 1,3-bis(2-hydroxyethoxy)benzene (0.025 mol), potassium carbonate (0.125 mol), tetrabutylammonium bromide (0.012 mol), and 40 ml of N,N-dimethylformamide were placed in a reactor equipped with a stirrer and a heater in a N₂ atmosphere and allowed to react at 130°C for 15 hours. After the reaction, the solution was added to 160 ml of water, and the precipitated solid was recovered by filtration. The solid was washed and dried to give an intermediate product M (nitro compound).

### Step (2a)

Next, the intermediate product M (0.1 mol), 5% palladium carbon (Pd/C) (1.0 g), and 200 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 600 ml of water to precipitate a solid. The solid was recovered and dried to give an intermediate product N (amine compound).

### Step (3b)

Next, the intermediate product N (0.025 mol), imidazole (0.1 mol), and 100 ml of acetonitrile were placed in a reactor equipped with a stirrer, a heater, and a dropping funnel in a N₂ atmosphere, and diphenyl phosphite (0.05 mol) was placed in the dropping funnel. After purging with carbon dioxide five times, diphenyl phosphite is slowly added dropwise with stirring at 25°C under constant supply of carbon dioxide to allow the mixture to react for 15 hours. After the reaction, the precipitated solid was recovered by filtration and washed to give an intermediate product O (urea compound).

### Step (4b)

Next, the intermediate product O (0.025 mol), p-toluenesulfonyl chloride (0.05 mol), and 40 ml of pyridine are placed in a reactor equipped with a stirrer in a N₂ atmosphere and stirred. The mixture is allowed to react at 25°C for 3 hours, and then 120 ml of methanol is added and further stirred at 25°C for 1 hour. The precipitated solid was recovered by filtration and washed to give CC4. The structure of CC4 was confirmed by NMR and IR.

### Example 15: Synthesis of Cyclic Carbodiimide CC5 (Scheme 1)

CC5: Mw = 296

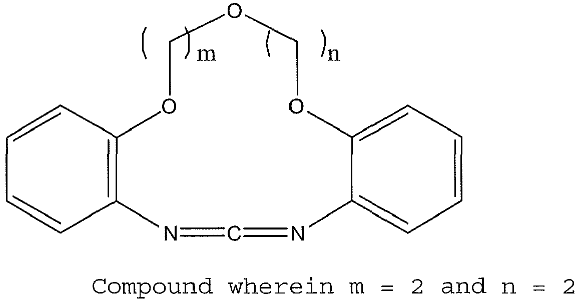

### Step (1b)

o-Chloronitrobenzene (0.0625 mol), diethylene glycol (0.025 mol), potassium carbonate (0.125 mol), tetrabutylammonium bromide (0.012 mol), and 40 ml of N,N-dimethylformamide were placed in a reactor equipped with a stirrer and a heater in a N₂ atmosphere and allowed to react at 130°C for 15 hours. After the reaction, the solution was added to 160 ml of water, and the precipitated solid was recovered by filtration. The solid was washed and dried to give an intermediate product P (nitro compound).

### Step (2a)

Next, the intermediate product P (0.1 mol), 5% palladium carbon (Pd/C) (1.0 g), and 150 ml of N,N-dimethylformamide were placed in a reactor equipped with a stirrer. The reactor was purged with hydrogen five times, and the mixture was allowed to react at 25°C under constant supply of hydrogen. The reaction is terminated when hydrogen stops decreasing. Pd/C is recovered by filtration, and the filtrate is placed in 450 ml of water to precipitate a solid. The solid was recovered and dried to give an intermediate product Q (amine compound).

### Step (3a)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product Q (0.05 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product R (triphenylphosphine compound).

### Step (4a)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product R (0.05 mol) is slowly added dropwise at 25°C. After dropping, the mixture is allowed to react for 12 hours. Subsequently, dichloromethane was removed, and the resulting solid was purified to give CC5. The structure of CC5 was confirmed by NMR and IR.

### Example 16: End-Capping of Polylactic Acid with CC3

0.005 parts by weight of tin octylate was added to 100 parts by weight of L-lactide (manufactured by Musashino Chemical Laboratory, optical purity: 100%), and the mixture was allowed to react in a nitrogen atmosphere in a reactor equipped with a stirring blade at 180°C for 2 hours. As a catalyst deactivator, phosphoric acid was added in an amount of 1.2 equivalents of tin octylate, then the residual lactide was removed at 13.3 Pa, and the resulting product was formed into chips to give poly(L-lactic acid). The obtained poly(L-lactic acid) had a carboxyl group concentration of 14 eq/ton.

100 parts by weight of the obtained poly(L-lactic acid) and 1.0 part by weight of CC3 were melt-kneaded in a twin-screw extruder (cylinder temperature: 230°C) for a residence time of 3 minutes. The carboxyl group concentration had decreased to 0.7 eq/ton or less. In addition, no isocyanate odor was detected at the outlet of the extruder after kneading.

### Example 17: End-Capping of Polylactic Acid with CC5

A reaction was carried out under the same conditions as in Example 16, except that the cyclic carbodiimide CC3 was replaced with the cyclic carbodiimide CC5. As a result, the carboxyl group concentration decreased to 0.4 eq/ton or less. In addition, no isocyanate odor was detected at the outlet of the extruder after kneading.

### Example 18: End-Capping of Polyamide with CC4

Poly(m-xylene adipamide) ("MX Nylon S6001" manufactured by Mitsubishi Gas Chemical), a polyamide made of m-xylylenediamine and adipic acid and having a carboxyl group concentration of 70 eq/ton, was used. 100 parts by weight of this poly(m-xylene adipamide) and 3.0 parts by weight of CC4 were melt-kneaded in a twin-screw extruder (cylinder temperature: 260°C) for a residence time of 3 minutes. The carboxyl group concentration had decreased to 1.9 eq/ton or less. In addition, no isocyanate odor was detected at the outlet of the extruder after kneading.

### Example19: Synthesis of Cyclic Carbodiimide CC6 (Scheme 1) CC6: MW = 324

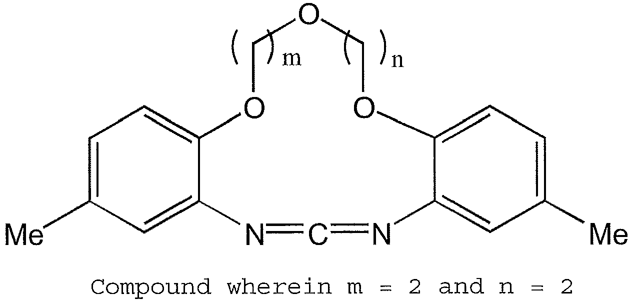

### Step (1b)

4-Chloro-3-nitrotoluene (0.11 mol), diethylene glycol (0.05 mol), potassium carbonate (0.33 mol), and 200 ml of N,N-dimethylformamide were placed in a reactor equipped with a stirrer and a heater in a N₂ atmosphere and allowed to react at 130°C for 12 hours. DMF was then removed under reduced pressure. The resulting solid was dissolved in 200 ml of dichloromethane, followed by partitioning with 100 ml of water three times. The organic layer was dried over 5 g of sodium sulfate, and dichloromethane was removed under reduced pressure to give an intermediate product P (nitro compound).

### Step (2a)

Next, the intermediate product P (0.1 mol), 5% palladium carbon (Pd/C) (1 g), and 200 ml of ethanol/dichloromethane (70/30) were placed in a reactor equipped with a stirrer. The reactor was purged with hydrogen five times, and the mixture was allowed to react at 25°C under constant supply of hydrogen. The reaction is terminated when hydrogen stops decreasing. Pd/C was recovered, and the mixed solvent was removed to give an intermediate product Q (amine compound).

### Step (3a)

Next, triphenylphosphine dibromide (0.11 mol) and 150 ml of 1,2-dichloroethane are placed in a reactor equipped with a stirrer, a heater, and a dropping funnel in a N₂ atmosphere and stirred. Then, a solution of the intermediate product Q (0.05 mol) and triethylamine (0.25 mol) dissolved in 50 ml of 1,2-dichloroethane is slowly added dropwise at 25°C. After the completion of dropping, the mixture is allowed to react at 70°C for 5 hours. Subsequently, the reaction solution was filtered, and the filtrate was partitioned with 100 ml of water five times. The organic layer was dried over 5 g of sodium sulfate, and 1,2-dichloroethane was removed under reduced pressure to give an intermediate product R (triphenylphosphine compound).

### Step (4a)

Next, di-tert-butyl dicarbonate (0.11 mol), N,N-dimethyl-4-aminopyridine (0.055 mol), and 150 ml of dichloromethane are placed in a reactor equipped with a stirrer and a dropping funnel in a N₂ atmosphere and stirred. Then, 100 ml of dichloromethane having dissolved therein the intermediate product R (0.05 mol) is slowly added dropwise at 25°C. After dropping, the mixture is allowed to react for 12 hours. Subsequently, dichloromethane was removed, and the resulting solid was purified to give CC6. The structure of CC6 was confirmed by NMR and IR.

## Claims

1. A cyclic carbodiimide compound represented by the following formula (i): 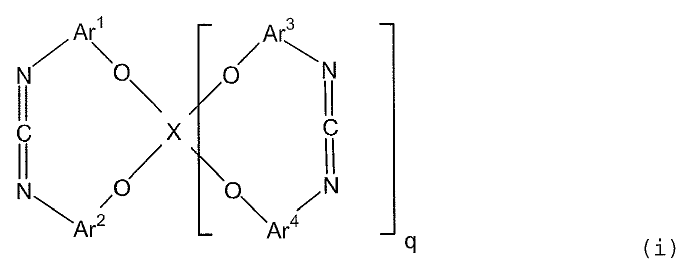 wherein
X is a divalent group represented by any one of the following formulae (i-1) to (i-6) or a tetravalent group represented by any one of the following formulae (i-7) and (i-8),
when X is a divalent group, q is 0, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
when X is a tetravalent group, q is 1, and Ar¹ to Ar⁴ are each independently an aromatic group substituted with a substituent other than a C₁₋₆ alkyl group and a phenyl group: 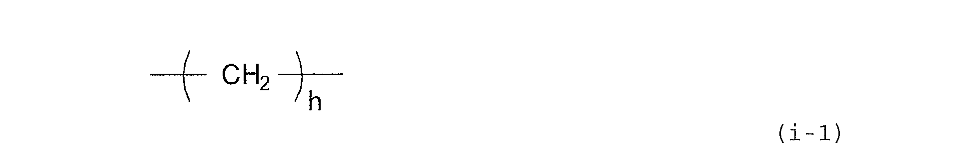 wherein h is an integer of 1 to 6, 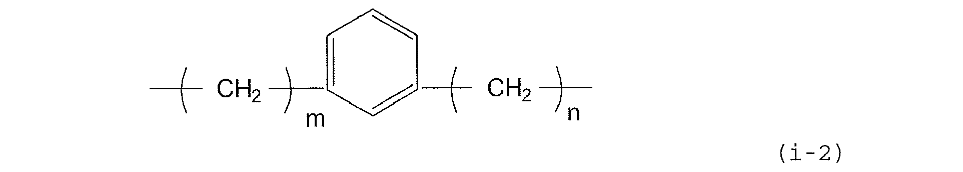 wherein m and n are each independently an integer of 0 to 3, 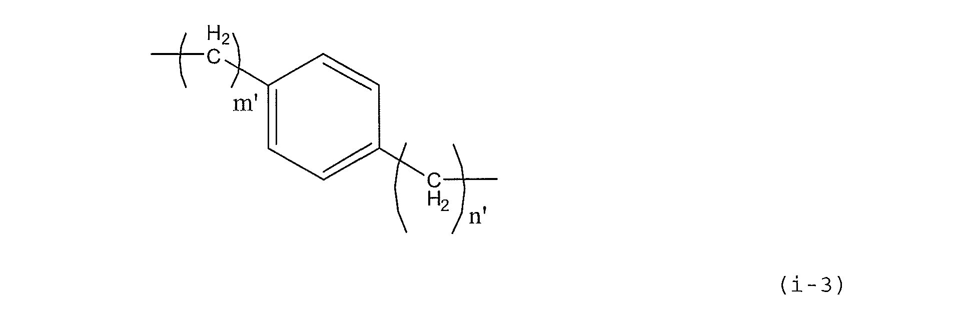 wherein m' and n' are each independently an integer of 0 to 3, 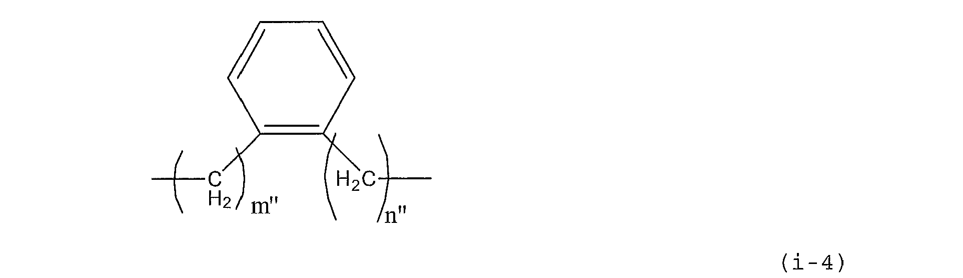 wherein m" and n" are each independently an integer of 0 to 3, 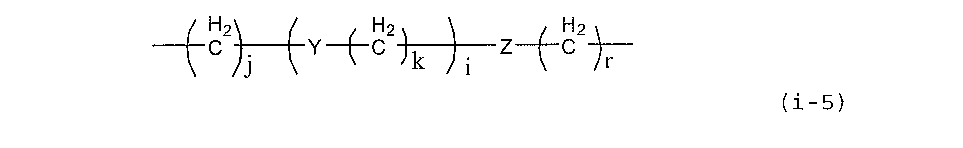 wherein Y and Z are each an oxygen atom or a sulfur atom, j, k, and r are each independently an integer of 1 to 4, and i is an integer of 0 to 3, 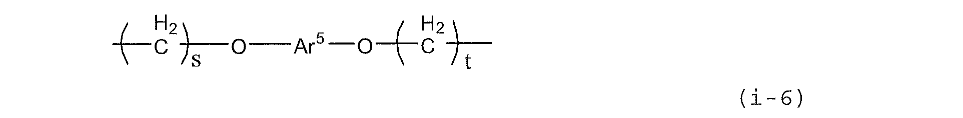 wherein Ar⁵ is an aromatic group, and s and t are each independently an integer of 1 to 3, 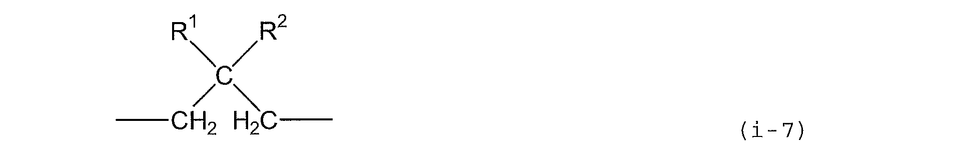 wherein R¹ and R² each independently represent a C₁₋₆ alkyl group or a phenyl group,

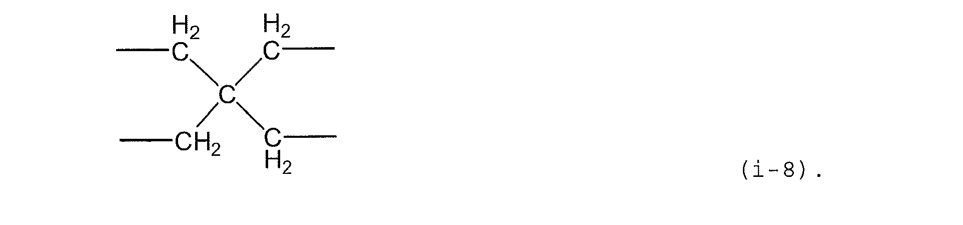

2. The compound according to claim 1, wherein Ar¹ to Ar⁴ are each independently an o-phenylene group or 1,2-naphthalene-diyl group substituted with a substituent other than a C₁₋₆ alkyl group and a phenyl group.

3. A method for producing the cyclic carbodiimide compound of claim 1, comprising:
(1) a step (1a) of allowing a compound of the following formula (a-1) and a compound of the following formula (a-2) to react with a compound of the following formula (b-1) to give a nitro compound of the following formula (c):
HO-Ar¹-NO₂ (a-1)
HO-Ar²-NO₂ (a-2)
E¹-X-E² (b-1) 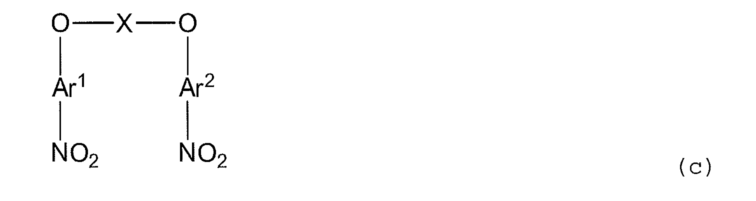
wherein
X, Ar¹, and Ar² are as defined in formula (i), with the proviso that X is a divalent group, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
E¹ and E² are each independently a group selected from the group consisting of a halogen atom, a toluenesulfonyloxy group, a methanesulfonyloxy group, a benzenesulfonyloxy group, and a p-bromobenzenesulfonyloxy group;
(2) a step (2a) of reducing the obtained nitro compound to give an amine compound represented by the following formula (d): 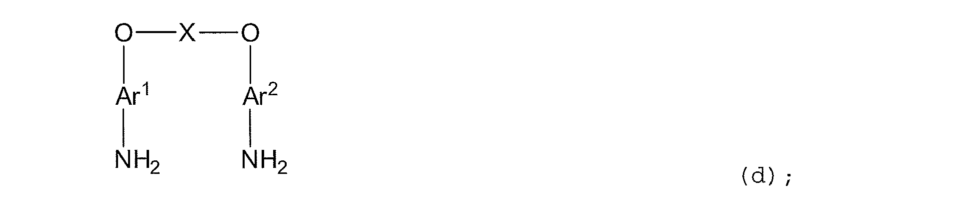
(3) a step (3a) of allowing the obtained amine compound to react with triphenylphosphine dibromide to give a triphenylphosphine compound represented by the following formula (e-1): 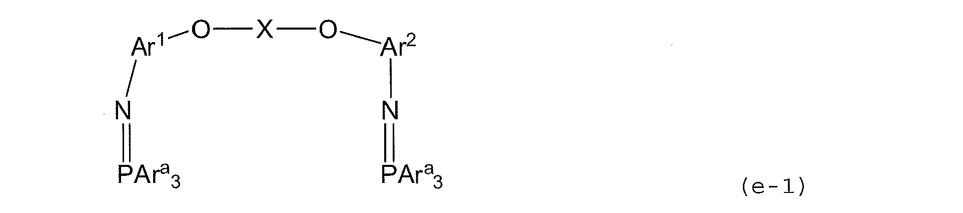 wherein Ar^{a} is a phenyl group; and
(4) a step (4a) of isocyanating the obtained triphenylphosphine compound in a reaction system, followed by direct decarboxylation to give a compound of the following formula (f):

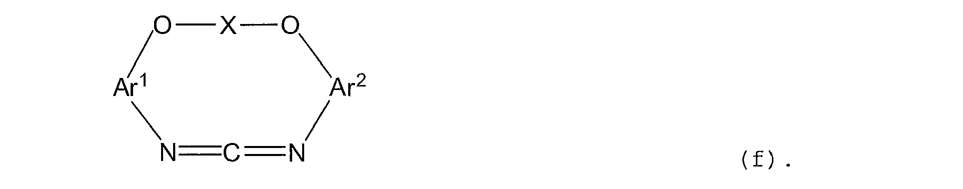

4. The method for producing the cyclic carbodiimide compound of claim 1 according to claim 3, wherein the step (1a) is replaced with a step (1b) of allowing a compound of the following formula (a-i) and a compound of the following formula (a-ii) to react with a compound of the following formula (b-i):
E³-Ar¹-NO₂ (a-i)
E⁴-Ar²-NO₂ (a-ii)
HO-X-OH (b-i)
wherein
X, Ar¹, and Ar² are as defined in formula (i), X is divalent, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
E³ and E⁴ are each independently a group selected from the group consisting of a halogen atom, a toluenesulfonyloxy group, a methanesulfonyloxy group, a benzenesulfonyloxy group, and a p-bromobenzenesulfonyloxy group.

5. The method for producing the cyclic carbodiimide compound of claim 1 according to claim 3, wherein
the step (3a) is replaced with a step (3b) of allowing the amine compound to react with carbon dioxide or carbon disulfide to give a urea compound or thiourea compound represented by the following formula (e-2): 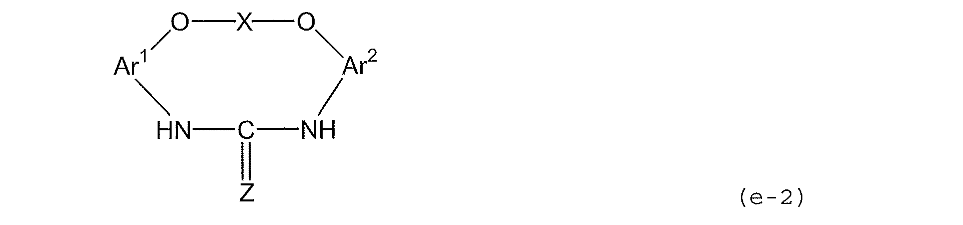 wherein
X, Ar¹, and Ar² are as defined in formula (i), X is divalent, and in the case where X is selected from (i-1) and (i-2), Ar¹ and Ar² are each independently an aromatic group substituted with a substituent other than a C₁₋₆ alkyl group and a phenyl group, while in the case where X is selected from (i-3) to (i-6), Ar¹ and Ar² are each independently an aromatic group optionally substituted with a substituent, and
Z is an oxygen atom or a sulfur atom, and
the step (4a) is replaced with a step (4b) of dehydrating the obtained urea compound or desulfurizing the obtained thiourea compound.

6. A method for producing the cyclic carbodiimide compound of claim 1, comprising:
(1) a step (1A) of allowing a compound of any one of the following formulae (A-1) to (A-4) to react with a compound of the following formula (B-1) to give a nitro compound of the following formula (C):
HO-Ar¹-NO₂ (A-1)
HO-Ar²-NO₂ (A-2)
HO-Ar³-NO₂ (A-3)
HO-Ar⁴-NO₂ (A-4)
wherein
Ar¹ to Ar⁴ are as defined in formula (i) and are each independently an aromatic group substituted with a substituent other than a C₁₋₆ alkyl group and a phenyl group, and
E¹ to E⁴ are each independently a group selected from the group consisting of a halogen atom, a toluenesulfonyloxy group, a methanesulfonyloxy group, a benzenesulfonyloxy group, and a p-bromobenzenesulfonyloxy group, 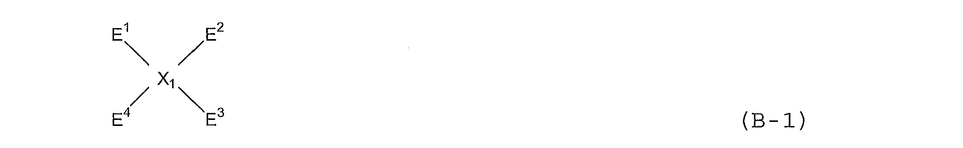 wherein X₁ is 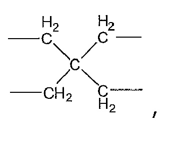 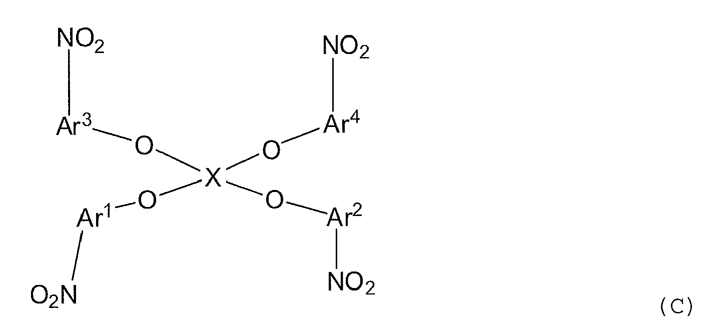 wherein X is as defined in formula (i), with the proviso that X is a tetravalent group represented by any one of formulae (i-7) and (i-8);
(2) a step (2A) of reducing the obtained nitro compound to give an amine compound of the following formula (D): 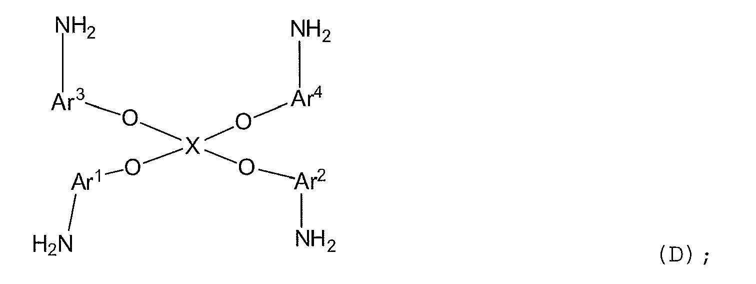
(3) a step (3A) of allowing the obtained amine compound to react with triphenylphosphine dibromide to give a triphenylphosphine compound of the following formula (E-1) : 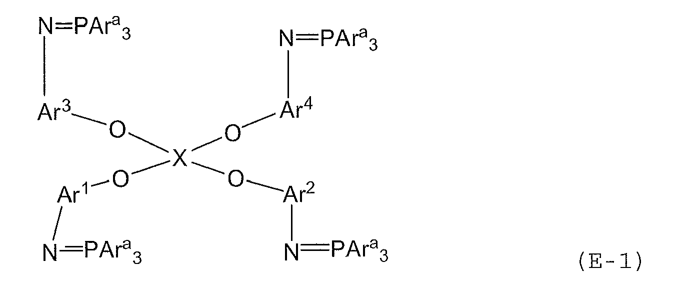 wherein Ar^{a} is a phenyl group; and
(4) a step (4A) of isocyanating the obtained triphenylphosphine compound in a reaction system, followed by direct decarboxylation to give a compound (F) of the following formula:

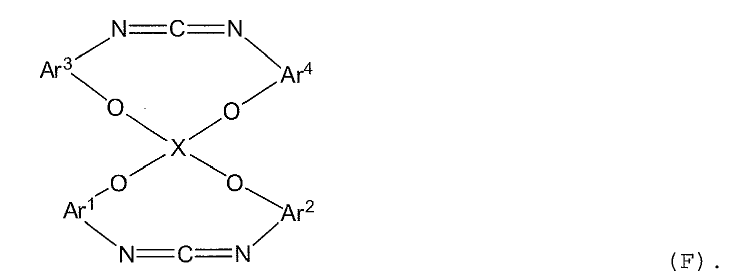

7. The method for producing the cyclic carbodiimide compound of claim 1 according to claim 6, wherein the step (1A) is replaced with a step (1B) of allowing a compound of any one of the following formulae (A-i) to (A-iv) to react with a compound of the following formula (B-i) to give a nitro compound of formula (C):
E⁵-Ar¹-NO₂ (A-i)
E⁶-Ar²-NO₂ (A-ii)
E⁷-Ar³-NO₂ (A-iii)
E⁸-Ar⁴-NO₂ (A-iv)
wherein
Ar¹ to Ar⁴ are as defined in formula (i) and are each independently an aromatic group substituted with a substituent other than a C₁₋₆ alkyl group and a phenyl group, and
E⁵ to E⁸ are each independently a group selected from the group consisting of a halogen atom, a toluenesulfonyloxy group, a methanesulfonyloxy group, a benzenesulfonyloxy group, and a p-bromobenzenesulfonyloxy group, 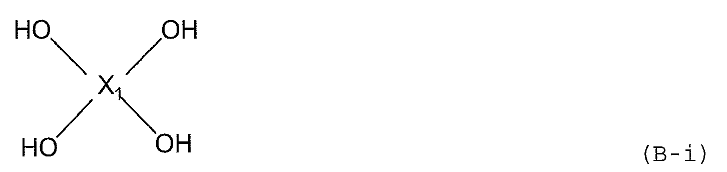 wherein X₁ is 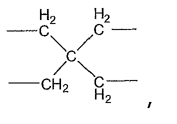 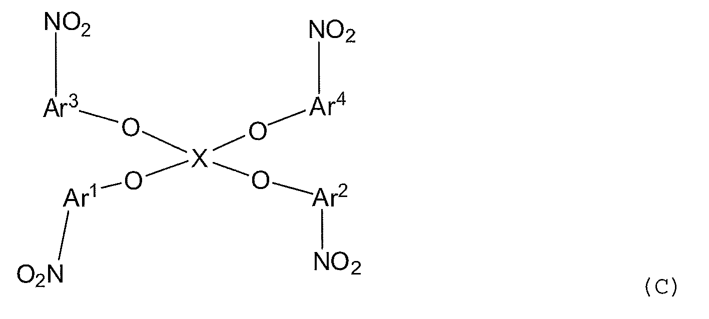 wherein X is as defined in formula (i), with the proviso that X is a tetravalent group represented by any one of formulae (i-7) and (i-8).

8. The method for producing the cyclic carbodiimide compound of claim 1 according to claim 6, wherein
the step (3A) is replaced with a step (3B) of allowing the amine compound to react with carbon dioxide or carbon disulfide to give a urea compound or thiourea compound of the following formula (E-2): 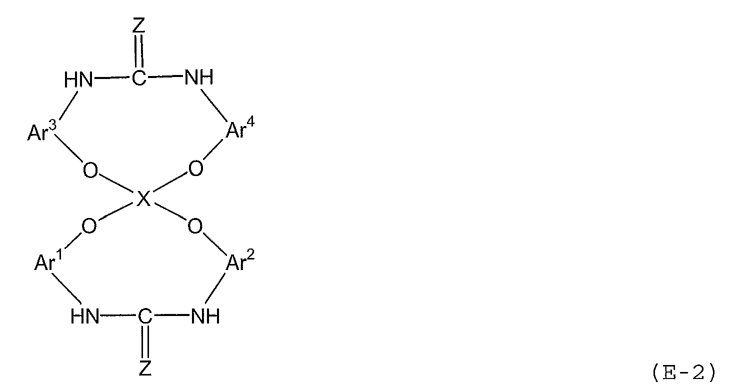 wherein
Ar¹ to Ar⁴ are as defined in formula (i) and are each independently an aromatic group substituted with a substituent other than a C₁₋₆ alkyl group and a phenyl group,
X is as defined in formula (i) and is a tetravalent group represented by any one of formulae (i-7) and (i-8), and
Z is an oxygen atom or a sulfur atom, and
the step (4A) is replaced with a step (4B) of dehydrating the obtained urea compound or desulfurizing the obtained thiourea compound.

9. An end-capping agent for polymer compounds, comprising the cyclic carbodiimide compound represented by formula (i) of claim 1 as an active ingredient.

10. An acidic group scavenger, comprising the cyclic carbodiimide compound represented by formula (i) of claim 1 as an active ingredient.
